# EUROPEAN PATENT SPECIFICATION

(11) **EP 4 561 978 B1**
(45) Date of publication and mention of the grant of the patent: **17.06.2026**
(21) Application number: 23750934.4
(22) Date of filing: 26.07.2023
(51) Int. Cl.: C07C 49/24, C07C 67/26, C07C 69/145

(54) **PROCESS FOR THE PREPARATION OF INTERMEDIATES IN FRAGRANCE PRODUCTION**
VERFAHREN ZUR HERSTELLUNG VON ZWISCHENPRODUKTEN IN DER DUFTSTOFFPRODUKTION
PROCÉDÉ DE PRÉPARATION DES INTERMÉDIAIRES DANS LA PRODUCTION DE PARFUMS

(30) Priority: 27.07.2022 GB 202210937
(43) Date of publication of application: 04.06.2025
(73) Proprietor: Givaudan SA, 1214 Vernier (CH)
(72) Inventor: GRANIER, Thierry, 8310 Kemptthal (CH); BAUMGARTNER, Corinne, 8310 Kemptthal (CH)
(74) Representative: Global Patents
(86) International application number: PCT/EP2023/070703
(87) International publication number: WO 2024/023154

(56) References cited:
- WO-A1-2021/209482
- HANUS JAN ET AL: "A Novel Method for Synthesis of cis -Zeatin and Its Valuable Precursor (Z) -4-Chloro-2-methyl-but-2-en-1-ol", ORGANIC PREPARATIONS AND PROCEDURES INTERNATIONAL: THE NEW JOURNAL FOR ORGANIC SYNTHESIS, vol. 51, no. 4, 10 May 2019 (2019-05-10), US, pages 368 - 374, XP093072648, ISSN: 0030-4948, DOI: 10.1080/00304948.2019.1609817

## Description

### TECHNICAL FIELD

The present invention relates to methods for making intermediates in the production of fragrances such as Amberketal and Amberketal homologues starting from β-farnesene. In particular, the invention relates to the production of acetylmethylfarnesol and hydroxy-farnesylacetone and homologues thereof. The invention further relates to a method for the production of intermediates thereof.

### BACKGROUND

Amberketal provides a powerful and tenacious ambery and woody odour that is useful in fragrance compositions alone or in combination with other woody or ambery ingredients. Amberketal is traditionally prepared from Manool via a number of chemical transformations. However, the supply of natural Manool is limited. It is therefore desirable to provide a new efficient and cost effective synthetic route to obtain Amberketal and Amberketal homologues. A synthetic route to Amberketal homologues (Formula A) comprising contacting a compound of Formula B (wherein R¹ is H, methyl or ethyl) with a squalene-hopene cyclase (SHC) enzyme or enzyme variant has been disclosed in WO2021/209482.

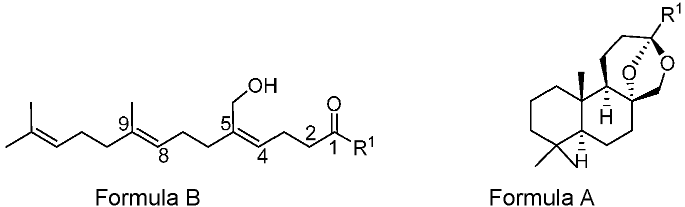

It is therefore desirable to provide a new efficient access to obtain compounds of Formula B, which can then be used in the production of Amberketal and Amberketal homologues.

### SUMMARY

In accordance with a first aspect of the present invention, there is provided a method of making a compound Formula (V) 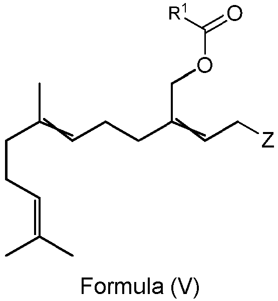 wherein the method comprises the step
a) contacting a mixture comprising a compound of Formula (III) and a compound of Formula (IV), with an acyl halogenide R¹C(O)X (e.g. acetyl chloride) optionally in the presence of halide salt (AY) 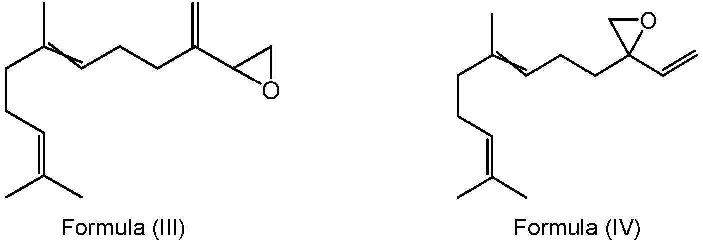 wherein R¹ is selected from the group consisting of C₁ to C₆ alkyl (such as ethyl, propyl, iso-propyl), X, Y and Z are selected from CI, I, and Br, and A is selected from Na, K, Li, and quaternary ammonium salts.

In a second aspect of the present invention, there is provided a compound of Formula (V) wherein R¹ is selected from the group consisting of C₁ to C₆ alkyl (such as ethyl, propyl, iso-propyl), and Z is selected from Cl, I, and Br.

In a third aspect of the present invention, there is provide a compound of Formula (VI) 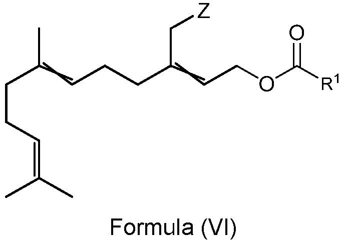 wherein R¹ is selected from the group consisting of C₁ to C₆ alkyl (such as ethyl, propyl, iso-propyl), and Z is selected from Cl, I, and Br.

Certain embodiments of any aspect of the present invention may provide one or more of the following advantages:
- the avoidance of expensive and less sustainable catalysts and ligands;
- controlling of the selectivity towards one isomer, if desired.
- a sequential one-pot synthesis without the direct isolation of unreacted starting material and/or intermediates (telescoped procedure).

The details, examples and preferences provided in relation to any particular one or more of the stated aspects of the present invention will be further described herein and apply equally to all aspects of the present invention. Any combination of the embodiments, examples and preferences described herein in all possible variations thereof is encompassed by the present invention unless otherwise indicated herein, or otherwise clearly contradicted by context.

### DETAILED DESCRIPTION

The present invention provides a novel and selective method for the production of a compound of Formula (I) via a mixture comprising an epoxide of Formula (III) and an epoxide of Formula (IV). The compound of Formula (I) may be used in the production of Amberketal and Amberketal homologues, as described, for example, in WO2021/209482.

In particular, the present invention is based, at least in part, on the surprising finding that the transformation of an epoxide (e.g. an epoxide of Formula (III), an epoxide of Formula (IV) as herein defined) can be performed avoiding the use of organometallic catalysts, such as homogenous transition metal catalysts (such as palladium, nickel, platinum, tungsten, iron, cobalt, cooper, molybdenum, ruthenium, rhodium, iridium and the like), which would be necessary for a Tsuji-Trost alkylation of 1,3-keto-esters with allylic epoxides and vinylic epoxides, such as the epoxides of Formula (IV) and Formula (III).

The inventors have found that the transformation of the epoxides is performed in the presence of an acyl halogenide as the activator.

Thus there is provided in a first aspect of the present invention a method for making a compound of Formula (V) 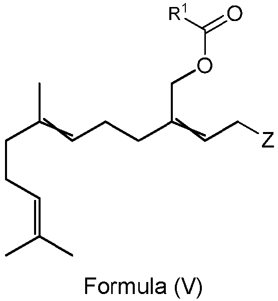 wherein the method comprises the step
a) contacting a mixture comprising a compound of Formula (III) and a compound of Formula (IV) with an acyl halogenide R¹C(O)X (e.g. acetyl chloride) optionally in the presence of halide salt (AY) 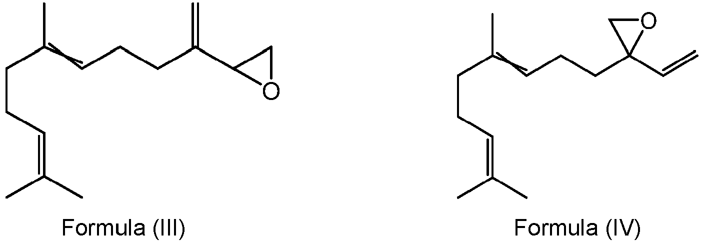 wherein R¹ is selected from the group consisting of C₁ to C₆ alkyl (such as ethyl, propyl, iso-propyl), X, Y, and Z are selected from Cl, I, and Br, and A is selected from Na, K, Li, and quaternary ammonium salts.

The epoxide of Formula (III) and the epoxide of Formula (IV) are in general obtained as a mixture, e.g., from β-farnesene (7,11-dimethyl-3-methylene-1,6,10-dodecatriene) as described in international patent application WO 2022/268840.

The inventors surprisingly have found that by using an acyl halogenide optionally in the presence of a halide salt (i.e. in the presence or absence of a halide salt), the ring-opening of the epoxide occurs either for both of the epoxides or only for one of the epoxides. This allows a chemical separation of the epoxide of Formula (III) and the epoxide of Formula (IV), if desired, as herein below described in more details.

If no halide salt is added to step a), the 1,4 halo-acylation was mainly observed for the exo-epoxide (compound of Formula (IV)) to form a compound of Formula (V). Surprisingly it was found that the 1,2-epoxide (compound of Formula (III)) is transformed more slowly into a compound of Formula (X) and its regio-isomer (X') 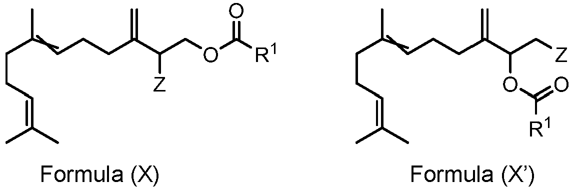 wherein R¹ is selected from the group consisting of C₁ to C₆ alkyl (such as ethyl, propyl, iso-propyl), Z is selected from Cl and Br,
both of which are formed via 1,2 addition of the acyl halogenide instead of the 1,4 addition, which was observed for the exo-epoxide, if at all.

The same was observed when a halide salt wherein the halide is not iodine was added to step a), namely mainly 1,4 halo-acylation for the exo-epoxide and more slowly transformation of the 1,2-epoxide.

When contacting a mixture comprising a compound of Formula (III) and a compound of Formula (IV) with an acyl halogenide R₁C(O)X wherein X is Cl or Br in the absent of a halide salt or in the presence of halide salt AY wherein the halide Y is not iodine (e.g. Y is Cl or Br), almost exclusively the (*Z*)-isomer of the compound of Formula (V), i.e. the compound of Formula (V') was formed. 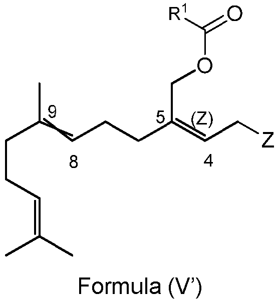 wherein R¹ is selected from the group consisting of C₁ to C₆ alkyl (such as ethyl, propyl, iso-propyl), Z is Cl or Br.

Thus there is provided in one particular embodiment of the first aspect of the present invention a method of making a compound of Formula (V') comprising the step
a) contacting a mixture comprising a compound of Formula (III) and a compound of Formula (IV) 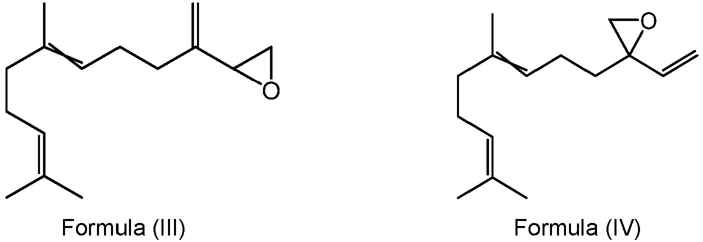
   i) with an acyl halogenide R¹C(O)X in the absent of a halide salt, or
   ii) with an acyl halogenide R¹C(O)X in the presence of halide salt AY wherein Y is not iodine (e.g. Y is selected from Cl and Br)
   wherein R¹ is selected from the group consisting of C₁ to C₆ alkyl (such as ethyl, propyl, iso-propyl), X is selected from Cl and Br, A is selected from Na, K, Li, and quaternary ammonium salts, and Z is selected from Cl and Br.

Without wishing to be bound by theory, it is thought that the halide **salt AY** (e.g. Y is selected from Cl and Br)
- might react with the acyl halogenide R¹C(O)X to form a new acyl halogenide R¹C(O)Y; and/or
- might react in combination with the acyl halogenide R¹C(O)X to open the epoxides.

If step a) is performed with an acyl halogenide R¹C(O)X in the presence of a iodine salt (AY wherein Y=I) it was observed that both epoxides, 1,2-epoxide and exo-epoxide, are transformed into the compound of Formula (V) and the compound of Formula (VI) respectively, in a similar speed, thus obtaining a mixture comprising a compound of Formula (V) and a compound of Formula (VI) 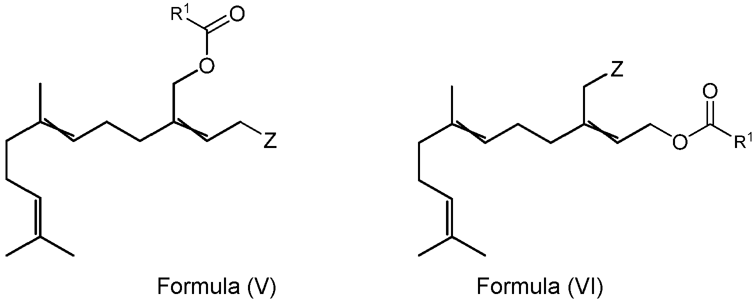 wherein
Z is iodine, and R¹ is selected from the group consisting of C₁ to C₆ alkyl (such as ethyl, propyl, iso-propyl).

The same was observed when step a) is performed with an acyl iodide (i.e. R¹C(O)X wherein X = I).

Thus there is provide in a further embodiment of the first aspect of the present invention a method of making a composition comprising compound of Formula (V) and a compound of Formula (VI) 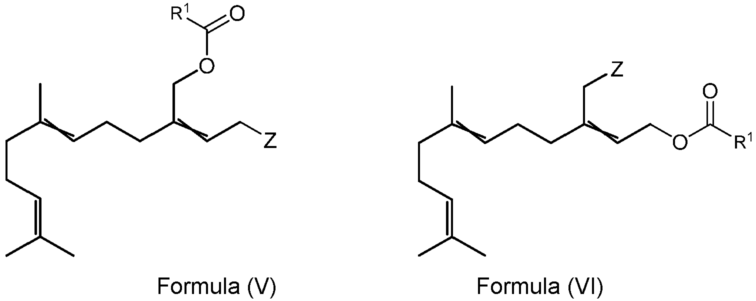 comprising the step
a) contacting a mixture comprising a compound of Formula (III) and a compound of Formula (IV) 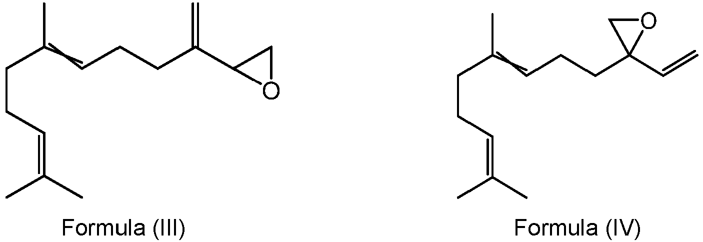 with an acyl iodide R₁C(O)I, or an acyl halogenide R₁C(O)X wherein X is selected from Cl and Br in the presence of iodine salt Al wherein A is selected from Na, K, Li, and quaternary ammonium salts, and wherein R¹ is selected from the group consisting of C₁ to C₆ alkyl (such as ethyl, propyl, iso-propyl), and Z is iodine.

The ratio of the compound of Formula (V) to the compound of Formula (VI) is maintained in the same ratio as the starting material, i.e. the ratio of the epoxides. For example, if the epoxides (III) and (IV) are present in a ratio of about 1 : 1, the compounds of Formula (V) and Formula (VI) will also be in the ratio of about 1 : 1.

The **acyl halogenide R¹C(O)X** present in step a) is in one particular embodiment selected from acyl chloride of the formula R¹C(O)Cl wherein R¹ is selected from the group consisting of C₁ to C₆ alkyl (such as ethyl, propyl, iso-propyl).

In some examples, the acyl halogenide R¹C(O)X present in step a) is selected from acetyl chloride and acetyl bromide.

In some examples, the acyl halogenide R¹C(O)X present in step a) is added in amount of about 0.9 to about 1.5 mol equivalents (e.g. about 1 to about 1.2 mol equivalents) in relation to the total amount of epoxides (compound of Formula (IV) and compound of Formula (III)).

In some examples, the acyl halogenide R¹C(O)X present in step a) is added in amount of about 0.9 to about 1.5 mol equivalents (e.g. about 1 to about 1.2 mol equivalents) in relation to the amount of compound of Formula (IV).

The **halide salt** if present in step a) is a compound AY wherein A is selected from Na, K, Li, and quaternary ammonium salts (for example, tetra-alkyl ammonium such as tetra-n-butylammonium), and Y is selected from CI, I, and Br, unless otherwise indicated.

In some examples, the halide salt present in step a) is selected from NaCl, Nal, NaBr, Lil, LiBr, Kl, and KBr.

In some examples, the halide salt present in step a) is tetra C₁-C₁₅ alkyl ammonium (e.g., tetra-n-butyl ammonium, n-methyl-tri-n-octyl ammonium).

In some examples, the halide salt if present in step a) is added in an amount of about 0.01 to about 1.2 mol equivalents for example, about 0.05 to about 1.1 mol equivalents, for example about 0.1 to about 1 mol equivalents, for example about 0.2 to about 0.9 mol equivalents, for example about 0.3 to about 0.8 mol equivalents, for example about 0.4 to about 0.7 mol equivalents, for example about 0.5 to about 0.6 mol equivalent in relation to the total amount of epoxides (compound of Formula (IV) and compound of Formula (III)).

In some examples, the halide salt if present in step a) is added in an amount of about 0.01 to about 1.2 mol equivalents, for example, about 0.05 to about 1.1 mol equivalents, for example about 0.1 to about 1 mol equivalents, for example about 0.2 to about 0.9 mol equivalents, for example about 0.3 to about 0.8 mol equivalents, for example about 0.4 to about 0.7 mol equivalents, for example about 0.5 to about 0.6 mol equivalent in relation to the amount of compound of Formula (IV).

In some examples, the method described hereinabove is performed at a **temperature** of room temperature or below, for example, from about 0°C to room temperature, or from about 5°C to room temperature, which includes 10°C. Room temperature may be a temperature of from about 20°C to about 25°C.

In some examples, the method described hereinabove is performed at room temperature (about 20°C to about 25°C) or above, for example, from about room temperature up to 120°C (e.g. up to 100°C or up to 70°C), which includes a temperature of about 25°C to about 65°C (e.g. about 50°C).

In some examples, the method described hereinabove is performed at a temperature which is the reflux temperature of the respective solvent used.

In some examples, the method described hereinabove is performed in the presence of a **solvent.** In some examples, the solvent is either a) ether solvent, such as dibutyl ether, methyl-tetrahydrofuran (Me-THF) and tetrahydrofuran (THF), or b) toluene, cyclohexane, or acetonitrile (MeCN).

In some examples, the method of making a compound of Formula (V'), a compound of Formula (X), and a compound of Formula (X'), or a mixture thereof comprises contacting a mixture comprising a compound of Formula (IV) and a compound of Formula (III) with
a) an acyl halogenide R¹C(O)X wherein X is Cl or Br in the absence of a halogenide salt, or
b) an acyl halogenide R¹C(O)X wherein X is Cl or Br in the presence of a halogenide salt AY wherein Y is Cl or Br and A is selected from Na, K, Li, and quaternary ammonium salts,
for from about 0.5h to about 24h, for example, from about 1h to about 18h, for example from about 1.5h to about 12h, for example from about 2h to about 8h, for example from about 3 h to about 4h.

Prolonged reaction time may result in forming a higher amount of the compound of Formula (X) and (X'). Thus the reaction time should be as short as possible. A good selectivity in favour of a compound of Formula (V') is obtained, for example, for from about 0.5 h to 5 h, for example from about 2h to about 4h.

In some examples, the compound of Formula (III) and the compound of Formula (IV) are *E-*Formula (III) and (IV).

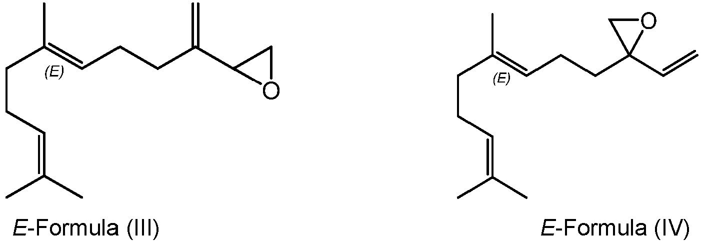

In some examples, the compound of Formula (III) is farnesene 1,2-epoxide, which is the compound with CAS number [83637-40-5].

In some examples, the compound of Formula (IV) is farnesene exo-epoxide, which is the compound with CAS number [1404220-65-0].

The thus obtained composition of the first aspect of the present invention comprising the compound of Formula (V) (which encompasses the compound of Formula (V')) may further be contacted (step b) with a compound of Formula (VII) 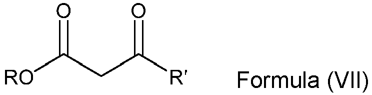 to obtain a compound of Formula (VIII) 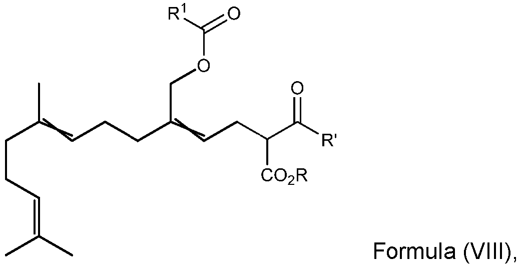 optionally followed by hydrolysis and decarboxylation of the compound of Formula (VIII) to obtain a compound of Formula (I) 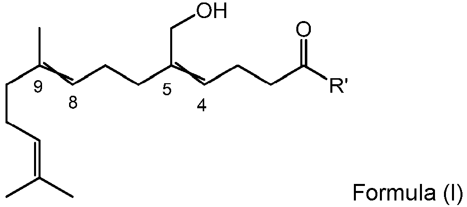 wherein R¹ is selected from the group consisting of C₁ to C₆ alkyl (such as ethyl, propyl, iso-propyl), R^{'} is selected from the group consisting of a C₁ - C₆ alkyl and an OR group, and R is selected from the group consisting of hydrogen and a C₁ to C₆ alkyl group.

As described herein above, it depends on the additives added to step a) and subsequent purification methods whether mainly a compound of Formula (V) (which encompasses the compound of Formula (V')) or a mixture comprising a compound of Formula (V) and a compound of Formula (VI) or a mixture comprising a compound of Formula (V') and a compound of Formula (X) and (X') is obtained.

Thus there is provided in a further embodiment of the present invention a method of making a compound of Formula (I) 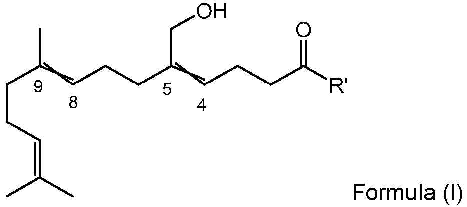 wherein the method comprises the steps
a) contacting a mixture comprising a compound of Formula (III) and a compound of Formula (IV) 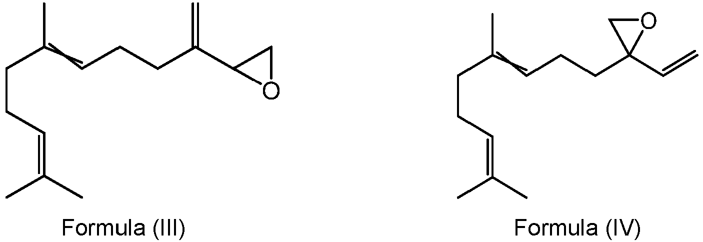
   i) with an acyl halogenide R₁C(O)X wherein X is selected from CI, I and Br in the absent of a halide salt, or
   ii) with an acyl halogenide R₁C(O)X wherein X is selected from CI, I and Br in the presence of halide salt AY wherein Y is not iodine (e.g. Y is selected from Cl and Br), and A is selected from Na, K, Li, and quaternary ammonium salts,
      to obtain a compound of Formula (V) 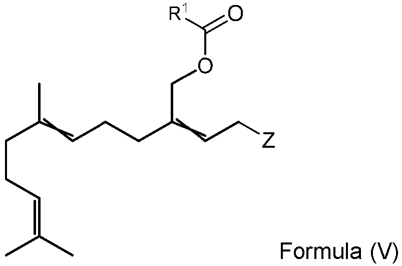
      wherein R¹ is selected from the group consisting of C₁ to C₆ alkyl (such as ethyl, propyl, iso-propyl) and Z is selected from CI, I and Br,
b) contacting the compound of Formula (V) with a compound of Formula (VII) 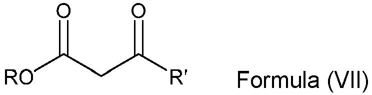 to obtain a compound of Formula (VIII) 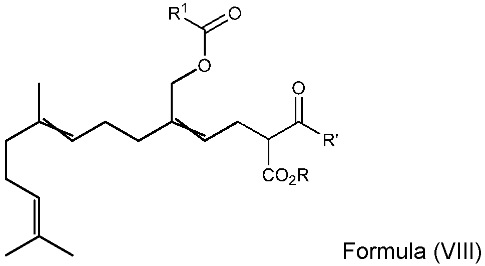 wherein R¹ is selected from the group consisting of C₁ to C₆ alkyl (such as ethyl, propyl, iso-propyl), R¹ is selected from the group consisting of a C₁ - C₆ alkyl and an OR group, and R is selected from the group consisting of hydrogen and a C₁ to C₆ alkyl group,
c) followed by hydrolysis and decarboxylation of the compound of Formula (VIII).

As already mentioned above,
a) in the presence of acyl halogenide R₁C(O)X wherein X is selected from Cl and Br and in the absent of a halide salt, or
b) in the presence of acyl halogenide R₁C(O)X wherein X is selected from Cl and Br and in the presence of a chloride salt or bromide salt (AY wherein Y is selected from Cl and Br and A has the meaning as provide herein above)
almost exclusively the (*Z*)-isomer of the compound of Formula (V), i.e. the compound of Formula (V') was formed, and thus almost exclusively the (*Z*)-isomer of the compound of Formula (VIII) (i.e. the compound of Formula (VIII')) is formed in step b) 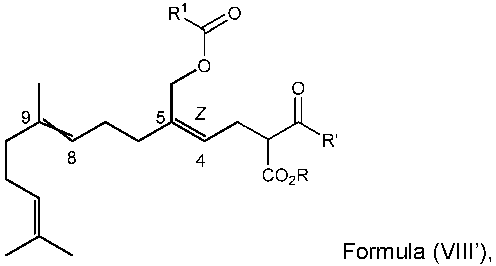 obtaining a compound of Formula (I') after hydrolysis and decarboxylation of the compound of Formula (VIII') (step c) 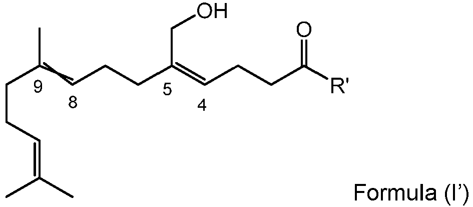 wherein R¹ is selected from the group consisting of C₁ to C₆ alkyl (such as ethyl, propyl, iso-propyl), R^{'} is selected from the group consisting of a C₁ - C₆ alkyl and an OR group, and R is selected from the group consisting of hydrogen and a C₁ to C₆ alkyl group.

This method has the advantage that the compound of Formula (I') can selectively be prepared from β-farnesene via the epoxide of Formula (IV), which allows an easier isolation of said compound. This method also allows for easier separation of either the compound of Formula (V'), the compound of Formula (VIII^{'}) or the compound of Formula (I') from the unreacted compound of Formula (III) or derivatives thereof. Consequently, higher overall yields are obtained.

In a further aspect of the present invention there is provide a method of making a mixture comprising a compound of Formula (I) and a compound of Formula (II) 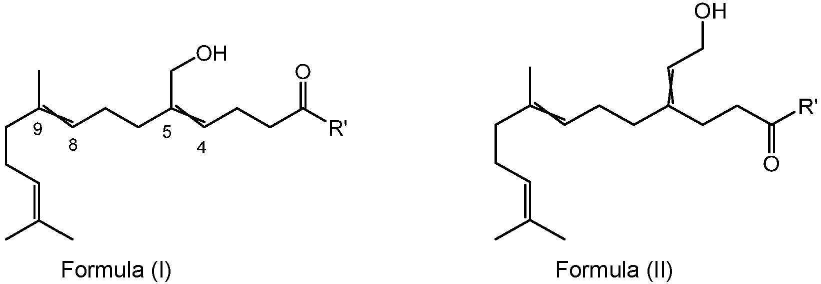 wherein the method comprises the steps
a) contacting a mixture comprising a compound of Formula (III) and a compound of Formula (IV) 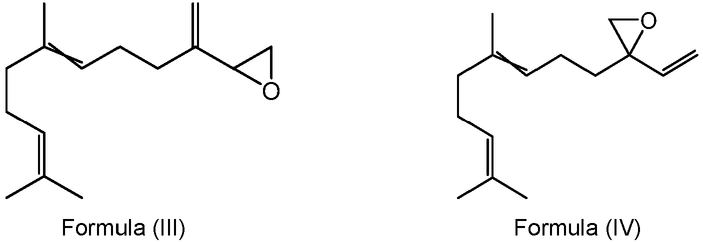 with
a) an acyl halogenide R¹C(O)X wherein X is selected from CI, I and Br in the presence of a halide salt AY wherein Y is iodine and A is selected from Na, K, Li, and quaternary ammonium salts, or
b) an acyl iodide (R¹C(O)X wherein X is I) in the absence of a halide salt to obtain a mixture comprising a compound of Formula (V) and a compound of Formula (VI) 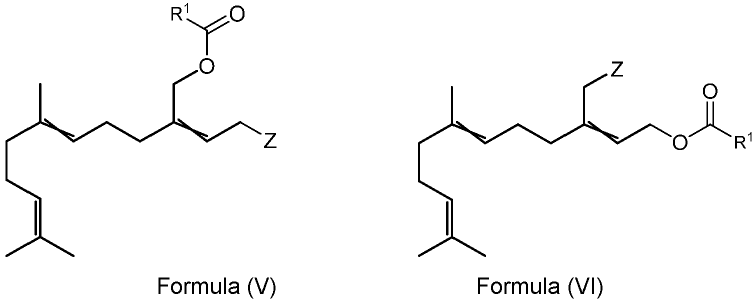 wherein
   Z is iodine, and R¹ is selected from the group consisting of C₁ to C₆ alkyl (such as ethyl, propyl, iso-propyl);
b) contacting the compound of Formula (V), the compound of Formula (VI) or a mixture thereof, respectively, with a compound of Formula (VII) to obtain a compound of Formula VIII, a compound of Formula (IX) or a mixture thereof 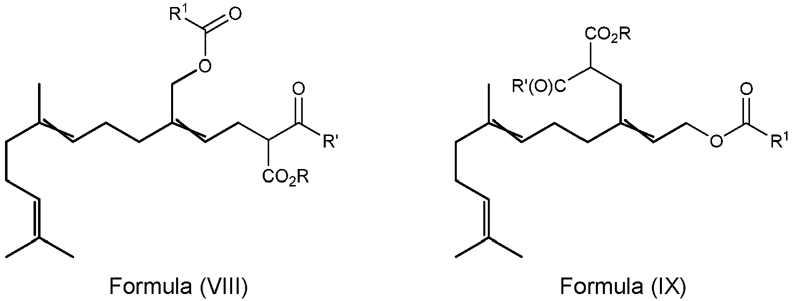 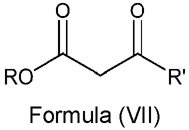 wherein
   R¹ is selected from the group consisting of C₁ to C₆ alkyl (such as ethyl, propyl, iso-propyl), R' is selected from the group consisting of a C₁ - C₆ alkyl and an OR group, and
   R is selected from the group consisting of hydrogen and a C₁ to C₆ alkyl group;
c) followed by hydrolysis and decarboxylation of the compound of Formula (VIII), the compound of Formula (IX) or a mixture thereof, respectively.

In one particular embodiment the method comprising the step a) to c) is to be understood as a sequential one-pot synthesis without the direct isolation of unreacted starting material and/or intermediates (telescoped procedure).

In another particular embodiment there is provided a method comprising the steps a) to c) characterised in that the obtained product of step a) (compound of Formula (V) and a compound of Formula (VI)) is purified first, followed by the method comprising the step b) and c) as a sequential one-pot synthesis.

In another particular embodiment there is provided a method comprising the steps a) to c) characterised in that the obtained product of step a) (compound of Formula (V')) is purified first, followed by the method comprising the step b) and c) as a sequential one-pot synthesis.

The compound of Formula (I) exists in the form of four different stereoisomers, for example, as a compound of Formula (I) having an *E,E*- or *E,Z*-configuration. The stereochemistry of the double bond between C-8 and C-9 remains unchanged during the method of making a compound of Formula (I), a compound of Formula (II) or a mixture thereof. In other word, if a compound of Formula (IV) wherein the double bond is in *E*-configuration is used as a starting material, the double bond between C-8 and C-9 of the compound of Formula (I) remains in *E*-configuration. If a compound of Formula (IV) wherein the double bond is in *Z*-configuration is used as a starting material, the double bound between C-8 and C-9 of the compound of Formula (I) remains in *Z*-configuration.

The same applies to the compound of Formula (II). In other word, if a compound of Formula (III) wherein the double bond is in *E*-configuration is used as a starting material, the double bond between C-8 and C-9 of the compound of Formula (II) remains in *E*-configuration. If a compound of Formula (III) wherein the double bond is in *Z*-configuration is used as a starting material, the double bound between C-8 and C-9 of the compound of Formula (II) remains in *Z*-configuration.

In one particular embodiment the compound of Formula (I) is obtained enriched in the isomer of Formula (I) wherein the double bond between C-4 and C-5 is in *Z*-configuration. By enriched we mean that the amount of the compound of Formula (I) wherein the double bond between C-4 and C-5 is in *Z*-configuration (Formula (I')) to the compound of Formula (I) wherein the double bond between C-4 and C-5 is in *E*-configuration is higher than 50% (for example the ratio between the *Z*-configuration compound to the *E*-configuration compound of Formula (I) is equal or greater than 51 : 49. For example, the weight ratio of the *Z-*configuration compound of Formula (I) to the *E*-configuration compound of Formula (I) is equal or greater than 2 : 1 (for example, 3 : 1 or greater, 4 : 1 or greater, 5 : 1 or greater, 6 : 1 or greater, 7 : 1 or greater, 8 : 1 or greater, 9: 1 or greater, 10 : 1 or greater).

In one particular example R¹ is methyl. When R¹ is methyl the compound of Formula (I) may be referred to as hydroxyl-farnesylacetone (6-(hydroxymethyl)-10,14-dimethylpentadeca-5,9,13-trien-2-one), encompassing *E,Z*-hydroxyfarnesylacetone (CAS 173198-97-5, i.e. a compound of Formula (I) wherein the double bond between C-4 and C-5 is in Z-configuration and the double bond between C-8 and C-9 is in *E*-configuration), *E,E-*hydroxyfarnesylacetone, *Z,Z*-hydroxyfarneslylacetone and *Z,E*-hydroxyfarnesylacetone. When R¹ is methyl the compound of Formula (II) may be referred to as acetylmethylfarnesol.

The compound of Formula (V) (which encompasses the compounds of Formula (V')) are not described in the literature and thus are novel in their own right.

Thus there is provided in a second aspect of the invention a compound of Formula (V) 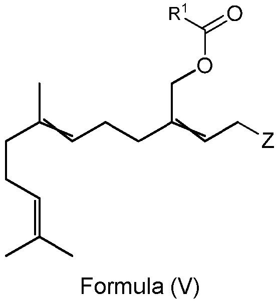 wherein R¹ is selected from the group consisting of C₄ to C₆ alkyl (such as ethyl, propyl, iso-propyl), and Z is selected from Cl, I, and Br.

The compound of Formula (VI) are not described in the literature and thus are novel in their own right.

Thus there is provided in a third aspect of the invention a compound of Formula (VI) 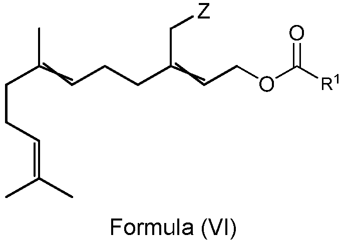 wherein R¹ is selected from the group consisting of C₁ to C₆ alkyl (such as ethyl, propyl, iso-propyl), and Z is selected from Cl, I, and Br.

The **compound of Formula (VII)** is an oxoalkanoic acid, an oxoalkanoic ester, malonic acid or a malonic ester. The compound of Formula (VII) may be an oxoalkanoic ester or a malonic ester.

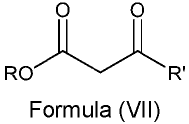

In some examples, R is selected from the group consisting of hydrogen and C₁ to C₆ alkyl groups. In some examples, R is selected from the group consisting of hydrogen, methyl, ethyl, propyl and isopropyl. In some examples, R is selected from the group consisting of hydrogen, methyl and ethyl. In some examples, R is methyl.

In some examples, R' is selected from the group consisting of a C₁ to C₆ alkyl group and an OR group. In some examples, R' is selected from the group consisting of a C₁ to C₆ alkyl group, hydroxyl and a C₁ to C₆ alkoxy group. In some examples, R' is selected from the group consisting of hydrogen, methyl, ethyl, propyl, isopropyl, hydroxyl, methoxy, ethoxy, propoxy and isopropoxy. In some examples, R' is selected from the group consisting of hydrogen, methyl, ethyl, propyl, isopropyl. In some examples, R' is hydroxyl or a methyl group. In some examples, R' is a methyl group.

In some examples, the compound of Formula (VII) is a compound of Formula (Vlla).

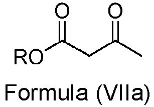

In some examples, a sodium salt of the compound of Formula (VII) (which encompasses the compound of Formula (Vlla)) is used. Said salts are commercially available, or they may be prepared following procedures well known to the person skilled in the art.

In some examples, step b) of the method herein described is performed in the presence of a base, in particular a non-nucleophilic base, such as potassium carbonate (K₂CO₃).

The examples described herein are illustrative of the present disclosure and are not intended to be limitations thereon. Different embodiments of the present disclosure have been described according to the present disclosure. Many modifications and variations may be made to the techniques described and illustrated herein without departing from the spirit and scope of the disclosure. Accordingly, it should be understood that the examples are illustrative only and are not limiting upon the scope of the disclosure.

### Examples

The following illustrates examples of the methods and other aspects described herein. Thus, these Examples should not be considered as limitations of the present disclosure, but are merely in place to teach how to make examples of the present disclosure.

### Example 1: (5Z,9E)-6-(hydroxymethyl)-10,14-dimethylpentadeca-5,9,13-trien-2-one

A solution of a mixture of beta farnesene, (*E*)-2-(4,8-dimethylnona-3,7-dien-1-yl)-2-vinyloxirane (*E*-Formula (IV)) and (*E*)-2-(6,10-dimethylundeca-1,5,9-trien-2-yl)oxirane *(E-*Formula (III)) (100 g; beta farnesene / *E*-Formula (IV) / *E*-Formula (III) = 1:0.7:0.5; 118 mmol *E*-Formula (IV)) in Me-THF (100 ml) at 5 °C was treated dropwise with acetyl chloride (10.2 ml, 142 mmol). The resulting mixture was stirred at 60 °C for 7h, at 50 °C for 16h and at 60 °C for 6h. After solvent evaporation under reduced pressure a crude mixture containing (2*Z*,5*E*)-2-(2-chloroethylidene)-6,10-dimethylundeca-5,9-dien-1-yl acetate (*E*-Formula (V'), Z=Cl) was isolated. The mixture was cooled to 5 °C and treated successively with Me-THF (65 ml), methyl acetoacetate (Formula (VII), R=Me, R'=Me; 28.3 ml, 260 mmol), Aliquat 336 (0.48 g, 1.18 mmol) and potassium carbonate (36.6 g, 260 mmol). The resulting mixture was stirred at 70 °C for 17h, cooled down to r.t. and treated successively with water (60 ml), and dropwise (keeping the internal temp. at 10°C) with 8M aq. NaOH solution (59 ml). The resulting biphasic mixture was vigorously stirred at r.t. for 3h and at reflux for 12h with subsequent addition of 8M aq. NaOH solution after 3h (50 ml).

The resulting mixture was cooled to r.t. and diluted with MTBE (30 ml) and water (75 ml). The aq. phase was extracted with MTBE (3 x 40 ml). The combined organic phases were washed with aq. saturated NH₄Cl solution (500 ml) and water (2 x 500 ml), dried over MgSO₄, filtered and concentrated under reduced pressure affording a crude orange oil containing beta farnesene, (*E*)-2-(4,8-dimethylnona-3,7-dien-1-yl)-2-vinyloxirane (*E*-Formula (IV)), (*E*)-2-(6,10-dimethylundeca-1,5,9-trien-2-yl)oxirane (*E*-Formula (III)) and (5*Z*,9*E*)-6-(hydroxymethyl)-10,14-dimethylpentadeca-5,9,13-trien-2-one (*E*,*Z*-Formula (I), R'=Me) (105.8 g; beta farnesene / *E*-Formula (IV) / *E*-Formula (III) / *E*,*Z*-Formula (I) = 1:0.1:0.5:0.5).

### (5Z,9E)-6-(hydroxymethyl)-10,14-dimethylpentadeca-5,9,13-trien-2-one:

¹H NMR (400 MHz, CDCl₃) *δ* ppm 5.17 (t, *J* = 7.6, 1H), 5.13-5.05 (m, 2H), 4.14 (s, 2H), 3.00-2.55 (br. s, OH), 2.55 (t, *J* = 6.7, 2H), 2.35 (dt, *J* = 6.9, 7.1, 2H), 2.13 (s, 3H, MeCO), 2.17-2.01 (m, 6H), 2.00-1.92 (m, 2H), 1.67 (br. s, 3H), 1.60 (br. s, 3H), 1.59 (br. s, 3H).

¹³C NMR (100 MHz, CDCl₃) *δ* ppm 209.02 (s, 1 C, C=O), 139.79 (s, 1 C), 135.30 (s, 1 C), 131.27 (s, 1 C), 126.65 (d, 1 C), 124.26 (d, 1 C), 123.85 (d, 1 C), 60.20 (t, 1 C), 43.19 (t, 1 C), 39.66 (t, 1 C), 35.60 (t, 1 C), 30.15 (q, 1 C), 26.74 (t, 1 C), 26.69 (t, 1 C), 25.66 (q, 1 C), 21.73 (t, 1 C), 17.65 (q, 1 C), 16.01 (q, 1 C).

GC-MS (El): 278 (1), 260 (1, [M-H₂O]^{+·}), 245 (1), 217 (3), 202 (1), 178 (5), 159 (4), 141 (5), 137 (4), 133 (19), 123 (13),105 (11), 95 (16), 93 (16), 81 (34), 79 (15), 69 (100), 67 (19), 55 (15), 43 (87), 41 (62), 31 (1).

### Example 2: (5Z,9E)-6-(hydroxymethyl)-10,14-dimethylpentadeca-5,9,13-trien-2-one

A solution of a mixture of beta farnesene, (*E*)-2-(4,8-dimethylnona-3,7-dien-1-yl)-2-vinyloxirane (*E*-Formula (IV)) and (*E*)-2-(6,10-dimethylundeca-1,5,9-trien-2-yl)oxirane (*E-*Formula (III)) (100 g; beta farnesene / *E*-Formula (IV) / *E*-Formula (III) = 1:0.65:0.45; 110 mmol *E*-Formula (IV)) in Me-THF (90 ml) at 5 °C was treated dropwise with acetyl chloride (9.5 ml, 132 mmol). The resulting mixture was stirred at 65 °C for 7h, at 50 °C for 15h and at 60 °C for 5h. The resulting solution was cooled to 5 °C and treated successively with methyl acetoacetate (Formula (VII), R=Me, R'=Me; 26.4 ml, 243 mmol), Aliquat 336 (0.45 g, 1.1 mmol) and potassium carbonate (34.2 g, 243 mmol). The resulting mixture was stirred at 70 °C for 15h, cooled down to r.t. and treated successively with water (60 ml), and dropwise (keeping the internal temp. at 10°C) with 50% aq. NaOH solution (44 g). The resulting biphasic mixture was vigorously stirred at r.t. for 1h and at reflux for 8h with subsequent addition of 50% aq. NaOH solution after 3h (23 ml).

The reaction mixture was cooled to r.t. and diluted with MTBE (30 ml) and water (75 ml). The aq. phase was extracted with MTBE (3 x 40 ml). The combined organic phases were washed with aq. saturated NH₄Cl solution (500 ml) and water (2 x 500 ml), dried over MgSO₄, filtered and concentrated under reduced pressure affording a crude orange oil containing beta farnesene, (*E*)-2-(4,8-dimethylnona-3,7-dien-1-yl)-2-vinyloxirane (*E*-Formula (IV)), (*E*)-2-(6,10-dimethylundeca-1,5,9-trien-2-yl)oxirane (*E*-Formula (III)) and (5*Z*,9*E*)-6-(hydroxymethyl)-10,14-dimethylpentadeca-5,9,13-trien-2-one (*E*,*Z*-Formula (I), R'=Me) (101.8 g; beta farnesene / *E*-Formula (IV) / *E*-Formula (III) / *E*,*Z*-Formula (I) = 1:0.04:0.45:0.41).

### Example 3: (2Z,5E)-2-(2-chloroethylidene)-6,10-dimethylundeca-5,9-dien-1-yl acetate

A solution of a mixture of beta farnesene, (*E*)-2-(4,8-dimethylnona-3,7-dien-1-yl)-2-vinyloxirane (*E*-Formula (IV)) and (*E*)-2-(6,10-dimethylundeca-1,5,9-trien-2-yl)oxirane (*E-*Formula (III)) (100 g; beta farnesene / *E*-Formula (IV) / *E*-Formula (III) = 1:0.9:0.6; 136 mmol *E*-Formula (IV)) in Me-THF (90 ml) at 10 °C was treated dropwise with acetyl chloride (12.1 ml, 170 mmol). The resulting mixture was stirred at 60 °C for 18h. After solvent evaporation under reduced pressure a crude mixture containing (2*Z*,5*E*)-2-(2-chloroethylidene)-6,10-dimethylundeca-5,9-dien-1-yl acetate (*E*-Formula (V'), Z=Cl) was isolated. The mixture was placed in a distillation apparatus and a polymeric amine (0.5 g) was added as a stabilizer. The mixture was short path distilled at 0.03 mbar to give a mixture of beta farnesene and (*E*)-2-(6,10-dimethylundeca-1,5,9-trien-2-yl)oxirane (*E*-Formula (III)) (58.1 g; beta farnesene / *E-*Formula (III) = 1:0.4) at a boiling point range of 88-93 °C and (2*Z*,5*E*)-2-(2-chloroethylidene)-6,10-dimethylundeca-5,9-dien-1-yl acetate (33.0 g, purity 75%, yield 59% / *E*-Formula (V'), Z=Cl) with a boiling point of 142 °C.

### (2Z,5E)-2-(2-chloroethylidene)-6,10-dimethylundeca-5,9-dien-1-yl acetate:

¹H NMR (400 MHz, CDCl₃) *δ* ppm 5.65 (br. t, *J* = 8.1, 1 H), 5.15-5.05 (m, 2H), 4.64 (s, 2H), 4.16 (d, *J* = 8.1, 2H), 2.06 (s, 3H), 2.25-1.95 (m, 8H), 1.68 (br. s, 3H), 1.61 (s, 6H).

¹³C NMR (100 MHz, CDCl₃) *δ* ppm 170.74 (s, 1 C), 139.54 (s, 1 C), 136.03 (s, 1 C), 131.36 (s, 1 C), 125.68 (d, 1 C), 124.21 (d, 1 C), 122.94 (d, 1 C), 61.18 (t, 1 C), 39.63 (t, 2 C), 35.10 (t, 1 C), 26.65 (t, 1 C), 26.16 (t, 1 C), 25.66 (q, 1 C), 20.88 (q, 1 C), 17.66 (q, 1 C), 16.04 (q, 1 C).

GC-MS (El): 298 (1), 262 (1), 239 (1), 223 (1), 203 (2), 195 (3), 159 (2), 137 (6), 133 (10), 123 (7), 105 (13), 93 (17), 91 (18), 81 (28), 69 (100), 79 (13), 67 (16), 55 (10), 53 (10), 43 (41), 41 (47).

### Example 4: (5Z,9E)-6-(hydroxymethyl)-10,14-dimethylpentadeca-5,9,13-trien-2-one

A solution of (2*Z*,5*E*)-2-(2-chloroethylidene)-6,10-dimethylundeca-5,9-dien-1-yl acetate (*E-*Formula (V'), Z=Cl, prepared according to Example 3; 40.0 g, 100 mmol), Aliquat 336 (0.34 g, 0.83 mmol) and methyl acetoacetate (Formula (VII), R=Me, R'=Me; 19 ml, 180 mmol) in Me-THF (40 ml) was cooled to 10 °C and treated with potassium carbonate (25 g, 180 mmol). The resulting mixture was stirred at 70 °C for 3h, cooled down to r.t. and treated successively with water (20 ml) and with 32% aq. NaOH solution (32 ml). The resulting mixture was stirred at reflux for 4h with subsequent addition of 32% aq. NaOH solution after 3h (21 ml).

The resulting mixture was cooled to r.t. and diluted with water (80 ml). The aq. phase was extracted with MTBE (100 ml). The combined organic phases were washed with aq. saturated NH₄Cl solution (200 ml), water (200 ml) and aq. saturated NaCl solution (200 ml), dried over MgSO₄, filtered and concentrated under reduced pressure affording (5Z,9E)-6-(hydroxymethyl)-10,14-dimethylpentadeca-5,9,13-trien-2-one (*E*,*Z*-Formula (I), R'=Me) (37.1 g purity 55%, yield 73%).

### Example 5: (2Z,5E)-2-(2-iodoethylidene)-6,10-dimethylundeca-5,9-dien-1-yl acetate and (2E,5E)-2-(2-iodoethylidene)-6,10-dimethylundeca-5,9-dien-1-yl acetate

A solution of (*E*)-2-(4,8-dimethylnona-3,7-dien-1-yl)-2-vinyloxirane (*E*-Formula (IV)) (0.30 g; 1.36 mmol) and Nal (0.21 g; 1.36 mmol) in MeCN (1 ml) at 5 °C was treated dropwise with acetyl chloride (0.1 ml, 1.43 mmol). The resulting mixture was stirred at 5 °C for 30min, diluted with EtOAc and water. The aq. phase was extracted with EtOAc (3 x). The combined organic phases were washed with aq. saturated NaCl solution, dried over MgSO₄, filtered and concentrated under reduced pressure affording a crude yellow oil containing (2*Z*,5*E*)-2-(2-iodoethylidene)-6,10-dimethylundeca-5,9-dien-1-yl acetate (*E*,*Z*-Formula (V), Z=I) and (2*E*,5*E*)-2-(2-iodoethylidene)-6,10-dimethylundeca-5,9-dien-1-yl acetate (*E*,*E*-Formula (V), Z=I) (0.46 g; *E,Z*-Formula (V) / *E*,*E*-Formula (V) = 3.5:1).

¹H-NMR (400 MHz, CDCl₃) *δ* ppm selected signals (*E,Z*-Formula (V)) 5.74 (br. t, *J* = 8.9, 1H), 5.18-5.03 (m, 2H), 4.63 (s, 2H, CH₂O), 3.96 (d, *J* = 8.9, 2H, CH₂I), 2.08 (s, 3H, Ac), 1.67 (s, 3H), 1.59 (s, 6H).

¹³C NMR (101 MHz, CDCl₃) δ ppm signals for (*E*,*Z*-Formula (V)) 170.83 (s, 1 C), 138.30 (s, 1 C), 135.88 (s, 1 C), 131.23 (s, 1 C), 127.34 (d, 1 C), 124.18 (d, 1 C), 122.86 (d, 1 C), 60.67 (t, 1 C), 39.56 (t, 1 C), 35.20 (t, 1 C), 26.57 (t, 1 C), 26.00 (t, 1 C), 25.61 (q, 1 C), 20.79 (q, 1 C), 17.62 (q, 1 C), 15.99 (q, 1 C), 0.19 (t, 1 C, CH₂I). 10-20% minor *E*,*E*-Formula (V).

### Example 6: (5Z,9E)-6-(hydroxymethyl)-10,14-dimethylpentadeca-5,9,13-trien-2-one (E,Z-Formula (I), R'=Me), (5E,9E)-6-(hydroxymethyl)-10,14-dimethylpentadeca-5,9,13-trien-2-one (E,E-Formula (I), R'=Me), (5Z,8E)-5-(2-hydroxyethylidene)-9,13-dimethyltetradeca-8,12-dien-2-one (E,Z-Formula (II), R'=Me) and (5E,8E)-5-(2-hydroxyethylidene)-9,13-dimethyltetradeca-8,12-dien-2-one (E,E-Formula (II), R'=Me)

A solution of a mixture of beta farnesene, (*E*)-2-(4,8-dimethylnona-3,7-dien-1-yl)-2-vinyloxirane (*E*-Formula (IV)) and (*E*)-2-(6,10-dimethylundeca-1,5,9-trien-2-yl)oxirane (*E-*Formula (III)) (0.5 g; beta farnesene / *E*-Formula (IV) / *E*-Formula (III) = 1:0.9:0.7; 1.18 mmol E-Formula (IV)) and Nal (0.18 g; 1.18 mmol) in MeCN (4 ml) at 5 °C was treated dropwise with acetyl chloride (0.09 ml, 1.24 mmol) and stirred at 5 °C for 2h. The resulting solution was treated successively with methyl acetoacetate (Formula (VII), R=Me, R'=Me; 0.26 ml, 2.36 mmol) and potassium carbonate (0.33 g, 2.36 mmol). The resulting mixture was stirred at 60 °C for 4h, cooled down to r.t. and diluted with MTBE and water. The aq. phase was extracted with MTBE (3 x). The combined organic phases were washed with aq. saturated NaCl solution, dried over MgSO₄, filtered and concentrated under reduced pressure affording a crude dark yellow oil containing a mixture of beta farnesene, (*E*,*Z-*Formula (VIII), R=R¹=R'=Me), (*E*,*E-*Formula (VIII), R=R¹=R'=Me), (*E*,*Z*-Formula (IX), R=R¹=R'=Me), and (*E*,*E*-Formula (IX), R=R¹=R'=Me) (0.66 g).

A mixture of the crude mixture from above and water (0.45 ml) was treated dropwise with 32% aq. NaOH solution (0.45 ml) and the resulting biphasic mixture was stirred at reflux for 1h. The reaction mixture was cooled to r.t. and diluted with MTBE and water. The aq. phase was extracted with MTBE (3). The combined organic phases were washed with aq. saturated NH₄Cl solution and aq. saturated NaCl solution (2 x), dried over MgSO₄, filtered and concentrated under reduced pressure affording 0.39 g of a crude orange oil containing 54% beta farnesene, 11% (*E*)-2-(6,10-dimethylundeca-1,5,9-trien-2-yl)oxirane (*E*-Formula (III)) and 35% of a mixture of (5*Z*,9*E*)-6-(hydroxymethyl)-10,14-dimethylpentadeca-5,9,13-trien-2-one (*E*,*Z-*Formula (I), R'=Me), (5*E*,9*E*)-6-(hydroxymethyl)-10,14-dimethylpentadeca-5,9,13-trien-2-one (*E*,*E*-Formula (I), R'=Me), (5*Z*,8*E*)-5-(2-hydroxyethylidene)-9,13-dimethyltetradeca-8,12-dien-2-one (*E*,*Z*-Formula (II), R'=Me) and (5*E*,8*E*)-5-(2-hydroxyethylidene)-9,13-dimethyltetradeca-8,12-dien-2-one (*E*,*E*-Formula (II), R'=Me) in a ratio of *E*,*Z*-Formula (I) / *E*,*E*-Formula (I) / *E*,*Z-*Formula (II) / *E*,*E*-Formula (II) = 5.5:1.0:3.3:1.9.

### (5Z,9E)-6-(hydroxymethyl)-10,14-dimethylpentadeca-5,9,13-trien-2-one: for analytical data see Example 1

(5*E*,9*E*)-6-(hydroxymethyl)-10,14-dimethylpentadeca-5,9,13-trien-2-one:
   ¹H NMR (400 MHz, CDCl₃) *δ* ppm 5.33 (t, *J* = 7.2, 1H), 5.13-5.01 (m, 2H), 3.98 (br. s, 2H), 2.46 (t, *J* = 7.3, 2H), 2.29 (dt, *J* = 7.1, 7.3, 2H), 2.22-2.05 (br. s, OH), 2.10 (s, 3H, MeCO), 2.13-1.98 (m, 6H), 1.97-1.90 (m, 2H), 1.64 (br. s, 3H), 1.56 (br. s, 6H).
   ¹³C NMR (100 MHz, CDCl₃) *δ* ppm 208.37 (s, 1 C), 139.91 (s, 1 C), 135.46 (s, 1 C), 131.19 (s, 1 C), 124.42 (d, 1 C), 124.11 (d, 1 C), 123.60 (d, 1 C), 66.54 (t, 1 C), 43.40 (t, 1 C), 39.57 (t, 1 C), 29.75 (q, 1 C), 27.96 (t, 1 C), 26.75 (t, 1 C), 26.51 (t, 1 C), 25.52 (q, 1 C), 21.68 (t, 1 C), 17.51 (q, 1 C), 15.84 (q, 1 C).
(5*Z*,8*E*)-5-(2-hydroxyethylidene)-9,13-dimethyltetradeca-8,12-dien-2-one:
   ¹H NMR (400 MHz, CDCl₃) *δ* ppm 5.45 (t, J = 7.2, 1H), 5.10-5.02 (m, 2H), 4.11 (d, J = 7.1, 2H), 2.53 (t, J = 7.4, 2H), 2.32 (t, J = 7.4, 2H), 2.11 (s, 3H, MeCO), 2.10-1.90 (m, 8H), 1.65 (br. s, 3H), 1.57 (s, 6H).
   ¹³C NMR (100 MHz, CDCl₃) *δ* ppm 208.47 (s, 1 C), 141.50 (s, 1 C), 135.45 (s, 1 C), 131.19 (s, 1 C), 124.81 (d, 1 C), 124.15 (d, 1 C), 123.42 (d, 1 C), 58.50 (t, 1 C), 41.89 (t, 1 C), 39.56 (t, 1 C), 36.14 (t, 1 C), 29.96 (q, 1 C), 26.57 (t, 1 C), 26.34 (t, 1 C), 25.56 (q, 1 C), 23.96 (t, 1 C), 17.55 (q, 1 C) 15.92 (q, 1 C).
(5*E*,8*E*)-5-(2-hydroxyethylidene)-9,13-dimethyltetradeca-8,12-dien-2-one:
   ¹H NMR (400 MHz, CDCl₃) *δ* ppm 5.35 (br. t, J = 6.9, 1H), 5.10-5.02 (m, 2H), 4.10 (d, J = 6.9, 2H), 2.57-2.52 (m, 2H), 2.28 (t, J = 7.1, 2H), 2.13 (s, 3H, MeCO), 2.10-1.90 (m, 8H), 1.65 (br. s, 3H), 1.57 (s, 6H).
   ¹³C NMR (100 MHz, CDCl₃) *δ* ppm 208.23 (s, 1 C), 141.48 (s, 1 C), 135.92 (s, 1 C), 131.28 (s, 1 C), 124.27 (d, 1 C), 124.08 (d, 1 C),123.25 (d, 1 C), 58.77 (t, 1 C), 41.93 (t, 1 C), 39.56 (t, 1 C), 30.64 (t, 1 C), 30.16 (t, 1 C), 29.80 (q, 1 C), 26.81 (t, 1C), 26.52 (t, 1 C), 25.56 (q, 1 C), 17.55 (q, 1 C), 15.89 (q, 1 C).

### Example 7: (E)-2-chloro-7,11-dimethyl-3-methylenedodeca-6,10-dien-1-yl acetate and (E)-1-chloro-7,11-dimethyl-3-methylenedodeca-6,10-dien-2-yl acetate

A solution of (*E*)-2-(6,10-dimethylundeca-1,5,9-trien-2-yl)oxirane (*E*-Formula (III)) (0.50 g; 2.1 mmol) in MeCN (3 ml) at 5 °C was treated dropwise with acetyl chloride (0.16 ml, 2.2 mmol). The resulting mixture was stirred at 5 °C for 3h and at r.t. for 24h and then diluted with MTBE and water. The aq. phase was extracted with MTBE (3x). The combined organic phases were washed with aq. saturated NaCl solution, dried over MgSO₄, filtered and concentrated under reduced pressure affording 0.62 g of a crude orange oil containing. a mixture of (*E*)-2-chloro-7,11-dimethyl-3-methylenedodeca-6,10-dien-1-yl acetate (*E*-Formula (X), Z=Cl) and (*E*)-1-chloro-7,11-dimethyl-3-methylenedodeca-6,10-dien-2-yl acetate (*E*-Formula (X'), Z=Cl) (*E-*Formula (X) / *E*-Formula (X') = 5.5:1) and an unidentified side product
(*E*)-2-chloro-7,11-dimethyl-3-methylenedodeca-6,10-dien-1-yl acetate:
   ¹H-NMR (500 MHz, CDCl₃) *δ* ppm 5.19 (br. s, 1H), 5.15-5.06 (m, 2H), 5.05 (br. m, 1H), 4.55 (br. t, *J* = 6.9*,* 1H, CHCl), 4.30 (d, *J* = 6.8, 2H, CH₂O), 2.23-1.94 (m, 8H), 2.07 (s, 3H), 1.67 (s, 3H), 1.61 (s, 3H), 1.59 (s, 3H).
   ¹³C NMR (125 MHz, CDCl₃) δ ppm 170.39 (s, 1 C), 144.91 (s, 1 C), 135.83 (s, 1 C), 131.24 (s, 1 C), 124.16 (d, 1 C), 123.16 (d, 1 C), 114.73 (t, 1 C), 65.86 (t, 1 C), 61.24 (d, 1 C), 39.57 (t, 1 C), 31.36 (t, 1 C), 26.55 (t, 1 C), 26.07 (t, 1 C), 25.60 (q, 1 C), 20.64 (q, 1 C), 17.59 (q, 1 C), 15.98 (q, 1 C).
   GC-MS (El): 298 (1), 283 (1), 238 (1), 223 (1), 203 (2), 137 (3), 133 (12), 123 (7), 105 (12), 93 (16), 91 (15), 81 (21), 69 (100), 79 (11), 67 (12), 55 (9), 53 (9), 43 (50), 41 (45).
(*E*)-1-chloro-7,11-dimethyl-3-methylenedodeca-6,10-dien-2-yl acetate:
   ¹H-NMR (500 MHz, CDCl₃) *δ* ppm 5.21 (br. s, 1H), 5.15-5.06 (m, 2H), 5.01 (br. m, 1H), 4.47 (dd, *J* = 5.5, 7.3, 1H, CHCl), 3.82 (dd, *J* = 5.3, 11.9, 1 H, CH₂O), 3.78 (dd, *J* = 7.5, 11.9, 1H, CH₂O), 2.28-1.94 (m, 8H), 2.07 (s, 3H), 1.67 (s, 3H), 1.61 (s, 3H), 1.59 (s, 3H).
   ¹³C NMR (125 MHz, CDCl₃) δ ppm (selected signals) 175.49 (s, 1C), 114.60 (t, 1 C) 66.34 (d, 1 C) 65.42 (t, 1 C).

### Example 8: (2Z,5E)-2-(2-bromoethylidene)-6,10-dimethylundeca-5,9-dien-1-yl acetate

A solution of a mixture of beta farnesene, (*E*)-2-(4,8-dimethylnona-3,7-dien-1-yl)-2-vinyloxirane (*E*-Formula (IV)) and (*E*)-2-(6,10-dimethylundeca-1,5,9-trien-2-yl)oxirane (*E-*Formula (III)) (1.0 g; beta farnesene / *E*-Formula (IV) / *E*-Formula (III) = 1:0.65:0.45; 1.1 mmol *E*-Formula (IV)) in MeCN (4 ml) at 5 °C was treated dropwise with acetyl bromide (0.1 ml, 1.3 mmol). The resulting mixture was stirred at 5 °C for 4h, diluted with MTBE and aq. saturated NH₄Cl solution. The aq. phase was extracted with MTBE (3 x). The combined organic phases were washed with aq. saturated NaCl solution, dried over MgSO₄, filtered and concentrated under reduced pressure affording a crude yellow oil containing beta farnesene, (*E*)-2-(4,8-dimethylnona-3,7-dien-1-yl)-2-vinyloxirane (*E*-Formula (IV)), (*E*)-2-(6,10-dimethylundeca-1,5,9-trien-2-yl)oxirane (*E*-Formula (III)) and (2*Z*,5*E*)-2-(2-bromoethylidene)-6,10-dimethylundeca-5,9-dien-1-yl acetate (*E*-Formula (V'), Z=Br) (1.07 g; beta farnesene / *E-*Formula (IV) / *E*-Formula (III) / *E*-Formula (V') = 1:0.06:0.31:0.21).

### (2Z,5E)-2-(2-bromoethylidene)-6,10-dimethylundeca-5,9-dien-1-yl acetate:

¹H-NMR (400 MHz, CDCl₃) *δ* ppm 5.74 (br. t, *J* = 8.6, 1 H), 5.15-5.05 (m, 2 H), 4.67 (s, 2 H, CH₂O), 4.06 (d, *J* = 8.6, 2 H, CH₂Br), 2.09 (s, Ac), 2.25-1.95 (m, 8 H), 1.69 (br. s, Me), 1.61 (s, 2 Me).

¹³C-NMR (100 MHz, CDCl₃) *δ* ppm 170.76 (s, 1 C), 139.93 (s, 1 C), 136.03 (s, 1 C), 131.36 (s, 1 C), 125.84 (d, 1 C), 124.23 (d, 1 C), 122.91 (d, 1 C), 60.95 (t, 1 C), 39.67 (t, 2 C), 35.20 (t, 1 C), 26.68 (t, 1 C), 26.14 (t, 1 C), 25.66 (q, 1 C), 20.87 (q, 1 C), 17.66 (q, 1 C), 16.05 (q, 1 C).

GC-MS (EI) m/z 291 (1), 249 (1), 224 (1), 203 (5), 177 (1), 161 (2), 147 (7), 137 (11), 133 (12), 123 (10), 119 (10), 105 (13), 93 (24), 91 (17), 81 (31), 79 (13), 69 (100), 67 (19), 55 (8), 53 (9), 43 (34), 41 (37), 28 (2).

### Example 9: (E)-2-bromo-7,11-dimethyl-3-methylenedodeca-6,10-dien-1-yl acetate and (2Z,5E)-2-(2-bromoethylidene)-6,10-dimethylundeca-5,9-dien-1-yl acetate

A solution of a mixture of beta farnesene, (E)-2-(4,8-dimethylnona-3,7-dien-1-yl)-2-vinyloxirane (*E*-Formula (IV)) and (*E*)-2-(6,10-dimethylundeca-1,5,9-trien-2-yl)oxirane (*E-*Formula (III)) (1.0 g; beta farnesene / *E*-Formula (IV) / *E*-Formula (III) = 1:0.65:0.45; 1.9 mmol *E*-Formula (IV) & *E*-Formula (III)) in MeCN (4 ml) at 5 °C was treated dropwise with acetyl bromide (0.15 ml, 1.9 mmol). The resulting mixture was stirred at 5 °C for 2h, diluted with MTBE and aq. saturated NH₄Cl solution. The aq. phase was extracted with MTBE (3 x). The combined organic phases were washed with aq. saturated NaCl solution, dried over MgSO₄, filtered and concentrated under reduced pressure affording a crude yellow oil containing beta farnesene, (*E*)-2-bromo-7,11-dimethyl-3-methylenedodeca-6,10-dien-1-yl acetate (*E*-Formula (X) , Z=Br) and (2*Z*,5*E*)-2-(2-bromoethylidene)-6,10-dimethylundeca-5,9-dien-1-yl acetate (*E-*Formula (V'), Z=Br) (1.07 g; beta farnesene / *E*-Formula (X) / *E*-Formula (V') = 1:0.27:0.28).

### (E)-2-bromo-7,11-dimethyl-3-methylenedodeca-6,10-dien-1-yl acetate:

¹H-NMR (500 MHz, CDCl₃) *δ* ppm selected signals 5.21 (br. s, 1 H), 5.15-5.05 (m, 2 H), 5.04 (br. m, 1 H), 4.65 (br. *t*, *J* = 7.3, 1 H, CHBr), 4.42 (dd, *J* = 7.3, 11.8, 1 H, CH₂O), 4.34 (dd, *J* = 7.3, 11.8, 1 H, CH₂O), 2.05 (s, Ac), 1.67 (s, Me), 1.61 (s, Me), 1.58 (s, Me).
¹³C-NMR (125 MHz, CDCl₃) *δ* ppm 170.24 (s, 1 C), 145.32 (s, 1 C), 135.80 (s, 1 C), 131.23 (s, 1 C), 124.15 (d, 1 C), 123.22 (d, 1 C), 123.15 (s, 1 C), 114.66 (t, 1 C), 65.61 (t, 1 C), 52.55 (d, 1 C), 39.55 (t, 1 C), 31.61 (t, 1 C), 26.54 (t, 1 C), 25.59 (q, 1 C), 20.61 (q, 1 C), 17.58 (q, 1 C), 15.98 (q, 1 C).
GC-MS (EI) m/z 282 (1), 203 (10), 161 (3), 147 (6), 135 (7), 133 (16), 123 (5), 119 (11), 107 (15), 105 (15), 95 (9), 93 (26), 91 (17), 81 (20), 79 (14), 69 (100), 67 (14), 55 (11), 53 (10), 43 (51), 41 (49), 29 (4).

### Example 10: (E)-2-bromo-7,11-dimethyl-3-methylenedodeca-6,10-dien-1-yl acetate, (E)-2-chloro-7,11-dimethyl-3-methylenedodeca-6,10-dien-1-yl acetate and (E)-1-bromo-7,11-dimethyl-3-methylenedodeca-6,10-dien-2-yl acetate

A solution of (*E*)-2-(6,10-dimethylundeca-1,5,9-trien-2-yl)oxirane (*E*-Formula (III)) (0.50 g; 2.1 mmol) and NaBr (0.22 g; 2.1 mmol) in MeCN (3 ml) at 5 °C was treated dropwise with acetyl chloride (0.16 ml, 2.2 mmol). The resulting mixture was stirred at 5 °C for 24h and at r.t. for 24h and then diluted with MTBE and water. The aq. phase was extracted with MTBE (3x). The combined organic phases were washed with aq. saturated NaCl solution, dried over MgSO₄, filtered and concentrated under reduced pressure affording 0.74 g of a crude orange oil containing a mixture of (*E*)-2-bromo-7,11-dimethyl-3-methylenedodeca-6,10-dien-1-yl acetate (*E*-Formula (X), Z=Br) (*E*)-2-chloro-7,11-dimethyl-3-methylenedodeca-6,10-dien-1-yl acetate (*E*-Formula (X), Z=Cl) and (*E*)-1-bromo-7,11-dimethyl-3-methylenedodeca-6,10-dien-2-yl acetate (*E*-Formula (X'), Z=Br) (*E*-Formula (X), Z=Br / *E*-Formula (X), Z=Cl / *E*-Formula (X') = 9:3.8:1).

### (E)-1-bromo-7,11-dimethyl-3-methylenedodeca-6,10-dien-2-yl acetate:

¹H-NMR (400 MHz, CDCl₃) *δ* ppm, selected signals: 5.25-4.95 (m, 4 H), 4.61 (br. dd, *J* = 6.0, 7.0, 1 H, CHBr), 3.88 (dd, *J* = 7.3, 11.9, 1 H, CH₂O), 3.84 (dd, *J* = 6.1, 11.9, 1 H, CH₂O).

### Example 11: (5Z,9E)-6-(hydroxymethyl)-10,14-dimethylpentadeca-5,9,13-trien-2-one

A solution of a mixture of beta farnesene, (*E*)-2-(4,8-dimethylnona-3,7-dien-1-yl)-2-vinyloxirane (*E*-Formula (IV)) and (*E*)-2-(6,10-dimethylundeca-1,5,9-trien-2-yl)oxirane (*E-*Formula (III)) (1.0 g; beta farnesene / *E*-Formula (IV) / *E*-Formula (III) = 1:0.65:0.45; 1.9 mmol *E*-Formula (IV) & E-Formula (III)) in MeCN (4 ml) at 5 °C was treated dropwise with acetyl bromide (0.15 ml, 2.0 mmol). The resulting mixture was stirred at 5 °C for 2h. After solvent evaporation under reduced pressure, the residual crude mixture was cooled to 5 °C and treated successively with THF (4 ml), methyl acetoacetate (Formula (VII), R=Me, R'=Me; 0.45 ml, 4.1 mmol) and potassium carbonate (0.58 g, 4.1 mmol). The resulting mixture was stirred at 70 °C for 3h, cooled down to r.t. and treated successively with water (1 ml), and dropwise (keeping the internal temp. at 10°C) with 8M aq. NaOH solution (1.4 ml). The resulting biphasic mixture was vigorously stirred at 75 °C for 3h.

The resulting mixture was cooled to r.t. and diluted with MTBE and water. The aq. phase was extracted with MTBE (3x). The combined organic phases were washed with aq. saturated NH₄Cl solution and water (2x), dried over MgSO₄, filtered and concentrated under reduced pressure affording a crude orange oil containing beta farnesene, (*E*)-2-(4,8-dimethylnona-3,7-dien-1-yl)-2-vinyloxirane (*E*-Formula (IV)), (*E*)-2-(6,10-dimethylundeca-1,5,9-trien-2-yl)oxirane (*E*-Formula (III)) and (5*Z*,9*E*)-6-(hydroxymethyl)-10,14-dimethylpentadeca-5,9,13-trien-2-one (*E*,*Z*-Formula (I), R'=Me) (1.07 g; beta farnesene / *E*-Formula (IV) / *E*-Formula (III) / *E*,*Z-*Formula (I) = 1:0.1:0.3:0.45).

### Example 12: (5Z,9E)-6-(hydroxymethyl)-10,14-dimethylpentadeca-5,9,13-trien-2-one

A solution of a mixture of beta farnesene, (*E*)-2-(4,8-dimethylnona-3,7-dien-1-yl)-2-vinyloxirane (*E*-Formula (IV)) and (*E*)-2-(6,10-dimethylundeca-1,5,9-trien-2-yl)oxirane (*E-*Formula (III)) (1.0 g; beta farnesene / *E*-Formula (IV) / *E*-Formula (III) = 1:0.65:0.45; 1.1 mmol *E*-Formula (IV)) in MeCN (4 ml) at 5 °C was treated dropwise with acetyl bromide (0.11 ml, 1.4 mmol). The resulting mixture was stirred at 5 °C for 5h and kept at this temperature overnight. After solvent evaporation under reduced pressure, the residual crude mixture was cooled to 5 °C and treated successively with THF (4 ml), methyl acetoacetate (Formula (VII), R=Me, R'=Me; 0.26 ml, 2.4 mmol) and potassium carbonate (0.34 g, 2.4 mmol). The resulting mixture was stirred at 70 °C for 3h, cooled down to r.t. and treated successively with water (0.4 ml), and dropwise (keeping the internal temp. at 10°C) with 8M aq. NaOH solution (0.8 ml). The resulting biphasic mixture was vigorously stirred at 75 °C for 3h.

The resulting mixture was cooled to r.t. and diluted with MTBE and water. The aq. phase was extracted with MTBE (3x). The combined organic phases were washed with aq. saturated NH₄Cl solution and water (2x), dried over MgSO₄, filtered and concentrated under reduced pressure affording a crude orange oil containing beta farnesene, (*E*)-2-(4,8-dimethylnona-3,7-dien-1-yl)-2-vinyloxirane (*E*-Formula (IV)), (*E*)-2-(6,10-dimethylundeca-1,5,9-trien-2-yl)oxirane (*E*-Formula (III)) and (5*Z*,9*E*)-6-(hydroxymethyl)-10,14-dimethylpentadeca-5,9,13-trien-2-one (*E*,*Z*-Formula (I), R'=Me) (1.02 g; beta farnesene / *E*-Formula (IV) / *E*-Formula (III) / *E*,*Z-*Formula (I) = 1:0.05:0.5:0.5).

### Example 13: (5Z,9E)-6-(hydroxymethyl)-10,14-dimethylpentadeca-5,9,13-trien-2-one

A solution of a mixture of beta farnesene, (*E*)-2-(4,8-dimethylnona-3,7-dien-1-yl)-2-vinyloxirane (*E*-Formula (IV)) and (*E*)-2-(6,10-dimethylundeca-1,5,9-trien-2-yl)oxirane (*E*-Formula (III)) ((1.0 g; beta farnesene / *E*-Formula (IV) / *E*-Formula (III) = 1:0.8:0.7; 2.3 mmol *E*-Formula (IV) & *E-*Formula (III)) and NaBr (0.24 g; 2.3 mmol) in MeCN (6 ml) at 5 °C was treated dropwise with acetyl chloride (0.17 ml, 2.4 mmol). The resulting mixture was stirred at 5 °C for 3.5h, then diluted with MTBE and water. The aq. phase was extracted with MTBE (3x). The combined organic phases were washed with aq. saturated NaCl solution, dried over MgSO₄, filtered and concentrated under reduced pressure affording 1.24g of a crude orange oil containing beta farnesene, (*E*)-2-bromo-7,11-dimethyl-3-methylenedodeca-6,10-dien-1-yl acetate (*E*-Formula (X) , Z=Br), (*E*)-1-bromo-7,11-dimethyl-3-methylenedodeca-6,10-dien-2-yl acetate (*E*-Formula (X'), Z=Br) and (2*Z*,5*E*)-2-(2-bromoethylidene)-6,10-dimethylundeca-5,9-dien-1-yl acetate (*E-*Formula (V'), Z=Br) (beta farnesene / *E*-Formula (X) / *E*-Formula (X') / *E*-Formula (V') = 1:0.26:0.05:0.27).

A solution of the above mixture in THF (6 ml) was treated successively with methyl acetoacetate (Formula (VII), R=Me, R'=Me; 0.36 ml, 3.3 mmol) and potassium carbonate (0.46 g, 3.3 mmol). The resulting mixture was stirred at 70 °C for 4h, cooled down first to r.t. and then treated at 5 °C successively with water (6 ml), and dropwise with 32% aq. NaOH solution (4 ml). The resulting biphasic mixture was vigorously stirred at 75 °C for 5h and at r.t. overnight. The resulting mixture was diluted with MTBE and water. The aq. phase was extracted with MTBE (3x). The combined organic phases were washed with aq. saturated NH₄Cl solution and aq. saturated NaCl solution (2x), dried over MgSO₄, filtered and concentrated under reduced pressure affording a crude orange oil containing beta farnesene, (*E*)-2-(6,10-dimethylundeca-1,5,9-trien-2-yl)oxirane (*E*-Formula (III)) and (5*Z*,9*E*)-6-(hydroxymethyl)-10,14-dimethylpentadeca-5,9,13-trien-2-one (*E*,*Z*-Formula (I), R'=Me) (0.99 g; beta farnesene / *E-*Formula (III) / *E*,*Z*-Formula (I) = 3.3:1:1.5).

### Example 14: (2Z,6E)-3-(iodomethyl)-7,11-dimethyldodeca-2,6,10-trien-1-yl acetate and (2E,6E)-3-(iodomethyl)-7,11-dimethyldodeca-2,6,10-trien-1-yl acetate

At 5°C, a mixture of (*E*)-2-(6,10-dimethylundeca-1,5,9-trien-2-yl)oxirane (*E*-Formula (III)) (0.2 g; 0.84 mmol), Nal (0.13 g, 0.84 mmol) and MeCN (1 ml) was treated dropwise with acetyl chloride (0.064 ml, 0.89 mmol), stirred at 5°C for 1h, diluted with EtOAc and poured into water. The aq. phase was extracted with EtOAc (3x) and the combined org. phases were washed with brine, dried over MgSO₄, filtered and concentrated under reduced pressure affording 0.31 g of a crude yellow oil containing (2*Z*,6*E*)-3-(iodomethyl)-7,11-dimethyldodeca-2,6,10-trien-1-yl acetate (*ZE*-Formula (VI), Z=I) and (2*E*,6*E*)-3-(iodomethyl)-7,11-dimethyldodeca-2,6,10-trien-1-yl acetate (*EE*-Formula (VI), Z=I) (*ZE*-Formula (VI) / *EE*-Formula (VI) = ~1:1).

### (6E)-3-(iodomethyl)-7,11-dimethyldodeca-2,6,10-trien-1-yl acetate:

¹H NMR (400 MHz, CDCl₃) *δ* ppm, selected signals 5.79 (t, *J* = 6.9, 1H), 5.51 (br. tt, *J* = 7.0, 1.2, 1H), 5.18-5.03 (m, 2 x 2H), 4.58 (d, *J* = 7.0, 2 H, CH₂O), 4.55 (d, *J* = 7.0, 2H, CH₂O), 3.95 (br. s, 2H, CH₂I), 3.94 (br. s, 2H, CH₂I), 2.35-1.95 (m), 2.07 (s, 3H), 2.06 (s, 3H), 1.68 (s, 2 x 3H), 1.60 (s, 2 x 3H).

¹³C NMR (100 MHz, CDCl₃) *δ* ppm 170.86 (s, 1 C), 170.81 (s, 1 C), 142.32 (s, 1 C), 142.16 (s, 1 C), 136.46 (s, 1 C), 136.06 (s, 1 C), 131.43 (s, 1 C), 131.35 (s, 1 C), 124.16 (d, 1 C), 124.10 (d, 1 C), 123.31 (d, 1 C), 122.89 (d, 1 C), 122.64 (d, 1 C), 122.40 (d, 1 C), 60.93 (t, 1 C), 60.23 (t, 1 C), 39.61 (t, 2 C), 35.47 (t, 1 C), 29.29 (t, 1 C), 26.63 (t, 1 C), 26.61 (t, 1 C), 26.54 (t, 1 C), 26.09 (t, 1 C), 25.66 (q, 2 C), 20.87 (q, 1 C), 20.85 (q, 1 C), 17.66 (q, 2 C), 16.07 (q, 1 C), 16.05 (q, 1 C) 11.47 (t, 1 C, CH₂I) 2.60 (t, 1 C, CH₂I).

### Example 15: (2Z,5E)-2-(2-iodoethylidene)-6,10-dimethylundeca-5,9-dien-1-yl acetate, (2E,5E)-2-(2-iodoethylidene)-6,10-dimethylundeca-5,9-dien-1-yl acetate, (2Z,6E)-3-(iodomethyl)-7,11-dimethyldodeca-2,6,10-trien-1-yl acetate and (2E,6E)-3-(iodomethyl)-7,11-dimethyldodeca-2,6,10-trien-1-yl acetate

At 5°C and protected from daylight, a solution of a mixture of beta farnesene, (*E*)-2-(4,8-dimethylnona-3,7-dien-1-yl)-2-vinyloxirane (*E*-Formula (IV)) and (*E*)-2-(6,10-dimethylundeca-1,5,9-trien-2-yl)oxirane (*E*-Formula (III)) (1.0 g; beta farnesene / *E*-Formula (IV) / *E*-Formula (III) = 1:0.65:0.45; 1.9 mmol *E*-Formula (IV) & *E*-Formula (III)) and MeCN (4 ml) was treated dropwise with acetyl iodide (0.16 ml, 2.0 mmol) and stirred at 5°C for 0.5h. The resulting solution was diluted with MTBE and aq. saturated NH₄Cl solution. The aq. phase was extracted with MTBE (3x) and the combined org. phases were washed with brine, dried (MgSO₄), filtered and concentrated under reduced pressure affording a crude dark orange oil (1.40 g) containing beta farnesene, (2*Z*,5*E*)-2-(2-iodoethylidene)-6,10-dimethylundeca-5,9-dien-1-yl acetate (*ZE-*Formula (V), Z=I) and (2*E*,5*E*)-2-(2-iodoethylidene)-6,10-dimethylundeca-5,9-dien-1-yl acetate (*EE*-Formula (V), Z=I) (*ZE*-Formula (V) / *EE*-Formula (V) = 4:1) as well as (2*Z*,6*E*)-3-(iodomethyl)-7,11-dimethyldodeca-2,6,10-trien-1-yl acetate (*ZE*-Formula (VI), Z=I) and (2*E*,6*E*)-3-(iodomethyl)-7,11-dimethyldodeca-2,6,10-trien-1-yl acetate (*EE*-Formula (VI), Z=I) (*ZE*-Formula (VI) / *EE*-Formula (VI) = 1:1) in a ratio of beta farnesene / *E*-Formula (V) / *E-*Formula (VI) = 1:0.39:0.44.

### Example 16: (5Z,9E)-6-(hydroxymethyl)-10,14-dimethylpentadeca-5,9,13-trien-2-one (E,Z-Formula (I), R'=Me), (5E,9E)-6-(hydroxymethyl)-10,14-dimethylpentadeca-5,9,13-trien-2-one (E,E-Formula (I), R'=Me), (5Z,8E)-5-(2-hydroxyethylidene)-9,13-dimethyltetradeca-8,12-dien-2-one (E,Z-Formula (II), R'=Me) and (5E,8E)-5-(2-hydroxyethylidene)-9,13-dimethyltetradeca-8,12-dien-2-one (E,E-Formula (II), R'=Me)

At 5°C and protected from daylight, a solution of a mixture of beta farnesene, (*E*)-2-(4,8-dimethylnona-3,7-dien-1-yl)-2-vinyloxirane (*E*-Formula (IV)) and (*E*)-2-(6,10-dimethylundeca-1,5,9-trien-2-yl)oxirane (*E*-Formula (III)) (1.0 g; beta farnesene / *E*-Formula (IV) / *E*-Formula (III) = 1:0.65:0.45; 1.9 mmol *E*-Formula (IV) & *E*-Formula (III)) and MeCN (4 ml) was treated dropwise with acetyl iodide (0.16 ml, 2.0 mmol) and stirred at 5°C for 0.5 h. The resulting solution was concentrated under reduced pressure, the residue was cooled to 5°C, dissolved in THF (4 ml) and treated successively with methyl acetoacetate (Formula (VII), R=Me, R'=Me; 0.45 ml, 4.1 mmol) and potassium carbonate (0.58 g, 4.1 mmol). The resulting mixture was was stirred at 66 °C for 6h and at r.t. overnight. The reaction mixture was cooled to 5 °C and treated dropwise with 32% aq. NaOH solution (1.4 ml, 15 mmol) and water (0.7 ml), and the resulting biphasic mixture was stirred at reflux for 3 h. The reaction mixture was cooled down to r.t. and diluted with MTBE and water. The aq. phase was extracted with MTBE (3x) and the combined org. phases were washed with aq. saturated NH₄Cl solution and with water (2x), dried over MgSO₄, filtered and concentrated under reduced pressure affording 0.993 g of a crude orange oil containing 50% beta farnesene and 40% of a mixture of (5*Z*,9*E*)-6-(hydroxymethyl)-10,14-dimethylpentadeca-5,9,13-trien-2-one (*E*,*Z*-Formula (I), R'=Me), (5*E*,9*E*)-6-(hydroxymethyl)-10,14-dimethylpentadeca-5,9,13-trien-2-one (*E*,*E*-Formula (I), R'=Me), (5*Z*,8*E*)-5-(2-hydroxyethylidene)-9,13-dimethyltetradeca-8,12-dien-2-one (*E*,*Z*-Formula (II), R'=Me) and (5*E*,8*E*)-5-(2-hydroxyethylidene)-9,13-dimethyltetradeca-8,12-dien-2-one (*E*,*E*-Formula (II), R'=Me) in a ratio of *E*,*Z*-Formula (I) / *E*,*E*-Formula (I) / *E*,*Z*-Formula (II) / *E*,*E*-Formula (II) = 5.5:1.2:3.3:1.6.

## Claims

1. A method of making a compound of Formula (V) 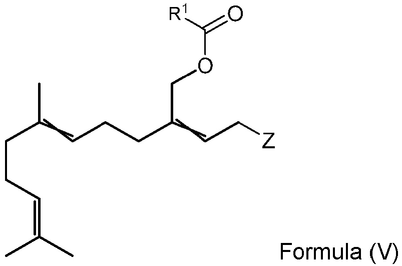 wherein the method comprises the step
a) contacting a mixture comprising a compound of Formula (III) and a compound of Formula (IV) with an acyl halogenide R¹C(O)X optionally in the presence of halide salt (AY) 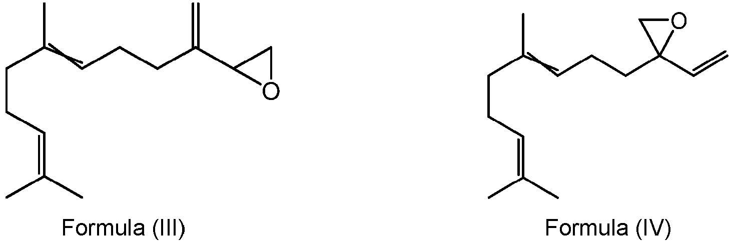 wherein R¹ is selected from the group consisting of C₁ to C₆ alkyl, X, Y and Z are selected from CI, I, and Br, and A is selected from Na, K, Li, and quaternary ammonium salts.

2. A method according to claim 1 of making a compound of Formula (V) wherein the compound of Formula (V) is a compound of Formula (V') 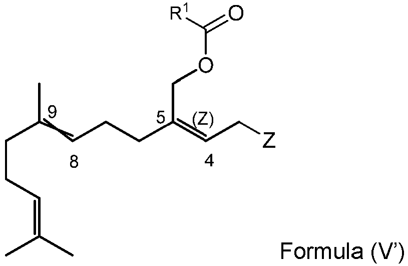 wherein the method comprises the step
a) contacting a mixture comprising a compound of Formula (III) and a compound of Formula (IV)
i) with an acyl halogenide R¹C(O)X in the absent of a halide salt, or
ii) with an acyl halogenide R¹C(O)X in the presence of halide salt AY wherein Y is not iodine,
wherein R¹ is selected from the group consisting of C₁ to C₆ alkyl, X is selected from Cl and Br, A is selected from Na, K, Li, and quaternary ammonium salts, and Z is selected from Cl and Br.

3. A method according to claim 2 of making a compound of Formula (V') and a compound of Formula (X) and/or its region-isomer (X') 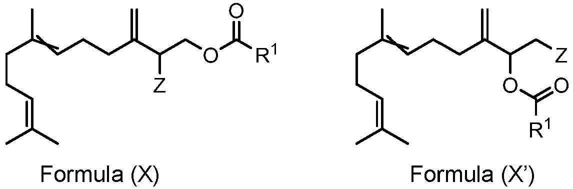 wherein R¹ is selected from the group consisting of C₁ to C₆ alkyl and Z is selected from Cl and Br.

4. A method according to claim 1 of making a compound of Formula (V) and a compound of Formula (VI) 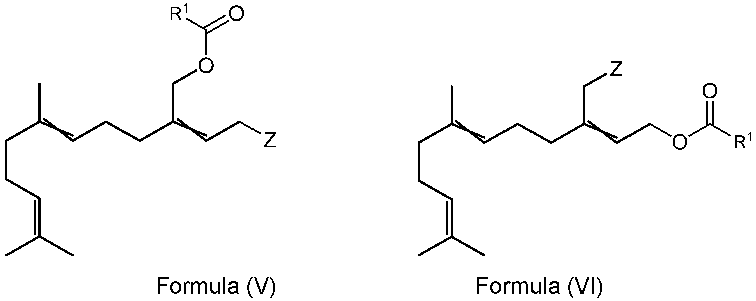 wherein the method comprises the step
a) contacting a mixture comprising a compound of Formula (III) and a compound of Formula (IV) with an acyl iodide (R₁C(O)I), or an acyl halogenide R₁C(O)X
wherein X is selected from Cl and Br in the presence of iodine salt Al wherein A is selected from Na, K, Li, and quaternary ammonium salts,
R¹ is selected from the group consisting of C₁ to C₆ alkyl, and Z is iodine.

5. A method according to any of claims 1 to 4 wherein the compound of Formula (III) and the compound of Formula (IV) are E-Formula (III) and (IV)

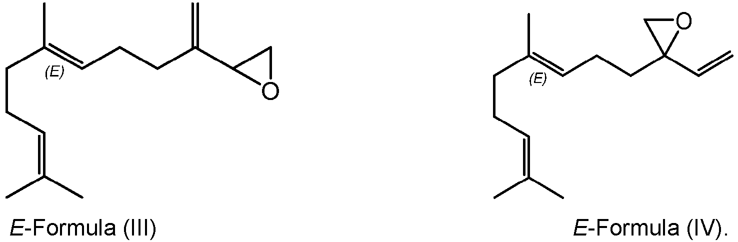

6. A method according to any of claims 1 to 5 followed by
step b) contacting a compound of Formula (V) with a compound of Formula (VII) 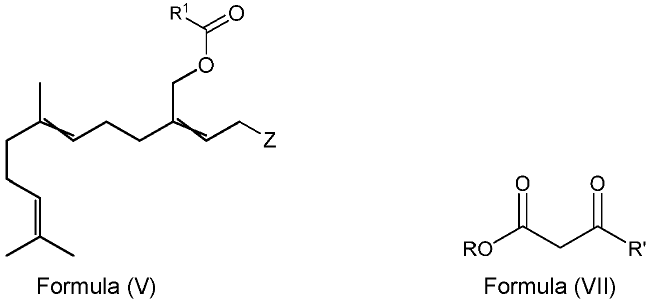 to obtain a compound of Formula (VIII) 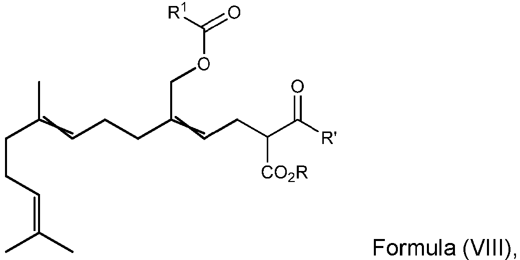 wherein R¹ is selected from the group consisting of C₁ to C₆ alkyl, R' is selected from the group consisting of a C₁ - C₆ alkyl and an OR group, R is selected from the group consisting of hydrogen and a C₁ to C₆ alkyl group, and Z is selected from CI, I and Br.

7. A method according to claim 6 followed by
step c) hydrolysis and decarboxylation of the compound of Formula (VIII) 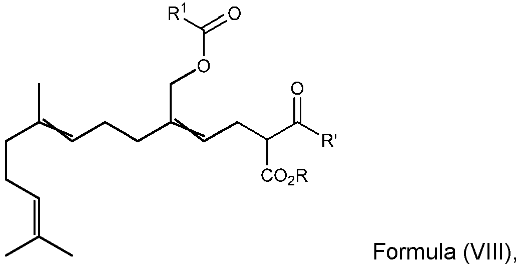 to obtain a compound of Formula (I) 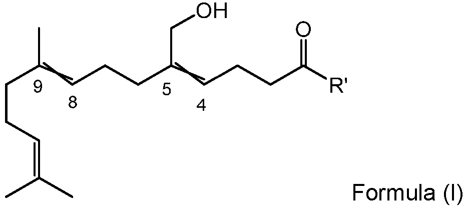 wherein R¹ is selected from the group consisting of C₁ to C₆ alkyl, R' is selected from the group consisting of a C₁ - C₆ alkyl and an OR group, and R is selected from the group consisting of hydrogen and a C₁ to C₆ alkyl group.

8. A method according to claim 7 wherein the step a) to c) is to be understood as a sequential one-pot synthesis.

9. A method according to claim 8 wherein the step a) to c) is to be understood as a sequential one-pot synthesis without the isolation of unreacted starting material.

10. A method according to claim 8 or claim 9 wherein the step a) to c) is to be understood as a sequential one-pot synthesis without the isolation of the intermediates.

11. A method according to claim 7 wherein the compound of Formula (V) obtained in step a) is purified first, followed by the method comprising the step b) and c) as sequential one-pot synthesis.

12. A method according to claim 11 wherein the compound of Formula (V) is a compound of Formula (V') 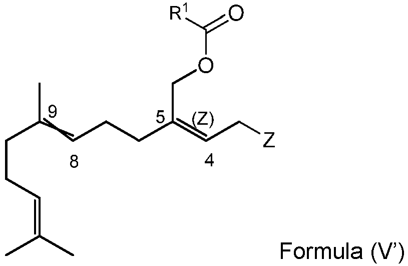 wherein
R¹ is selected from the group consisting of C₁ to C₆ alkyl, and Z is selected from Cl and Br.

13. A method according to any of claims 1 to 12 wherein R¹ is methyl.

14. A compound of Formula (V) 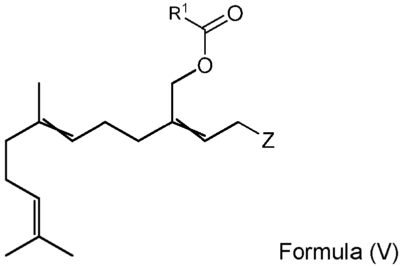 wherein
R¹ is selected from the group consisting of C₁ to C₆ alkyl and Z is selected from CI, I, and Br.

15. A compound of Formula (VI) 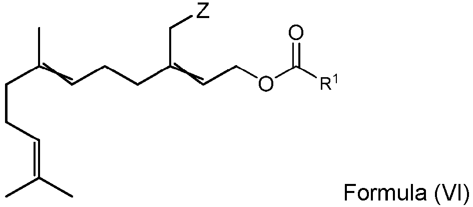 wherein
R¹ is selected from the group consisting of C₁ to C₆ alkyl and Z is selected from Cl, I, and Br.

## Patentansprüche

1. Verfahren zur Herstellung einer Verbindung der Formel (V) 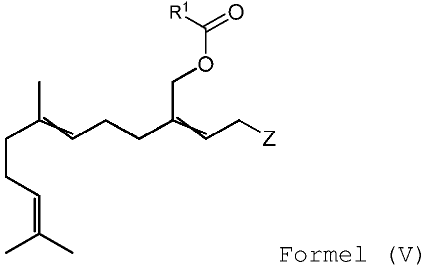
wobei das Verfahren den folgenden Schritt umfasst:
a) Inkontaktbringen einer Mischung, die eine Verbindung der Formel (III) und eine Verbindung der Formel (IV) umfasst, mit einem Acylhalogenid R¹C(O)X, gegebenenfalls in Gegenwart von Halogenidsalz (AY), 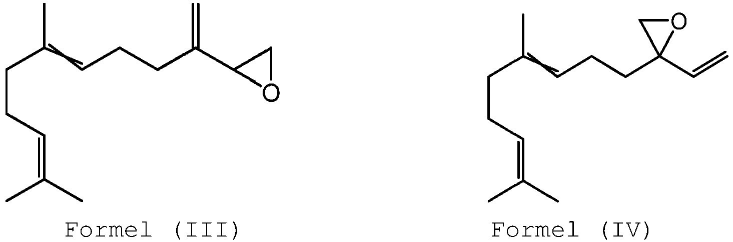
wobei R¹ aus der Gruppe bestehend aus C₁- bis C₆-Alkyl ausgewählt ist, X, Y und Z aus Cl, I und Br ausgewählt sind und A aus Na, K, Li und quartären Ammoniumsalzen ausgewählt ist.

2. Verfahren nach Anspruch 1 zur Herstellung einer Verbindung der Formel (V), wobei es sich bei der Verbindung der Formel (V) um eine Verbindung der Formel (V') handelt: 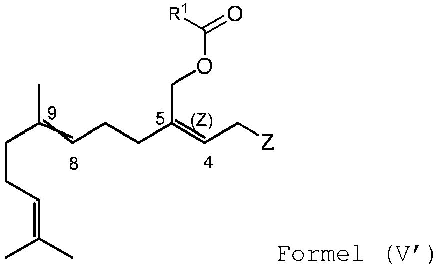
wobei das Verfahren den folgenden Schritt umfasst:
a) Inkontaktbringen einer Mischung, die eine Verbindung der Formel (III) und eine Verbindung der Formel (IV) umfasst,
i) mit einem Acylhalogenid R¹C(O)X in Abwesenheit eines Halogenidsalzes oder
ii) mit einem Acylhalogenid R¹C(O)X in Gegenwart von Halogenidsalz AY, wobei Y nicht für Iod steht,
wobei R¹ aus der Gruppe bestehend aus C₁- bis C₆-Alkyl ausgewählt ist, X aus Cl und Br ausgewählt ist, A aus Na, K, Li und quartären Ammoniumsalzen ausgewählt ist und Z aus Cl und Br ausgewählt ist.

3. Verfahren nach Anspruch 2 zur Herstellung einer Verbindung der Formel (V') und einer Verbindung der Formel (X) und/oder ihres Regioisomers (X') 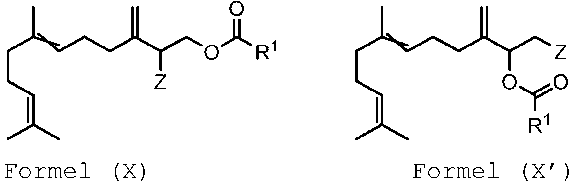 wobei R¹ aus der Gruppe bestehend aus C₁- bis C₆-Alkyl ausgewählt ist und Z aus Cl und Br ausgewählt ist.

4. Verfahren nach Anspruch 1 zur Herstellung einer Verbindung der Formel (V) und einer Verbindung der Formel (VI) 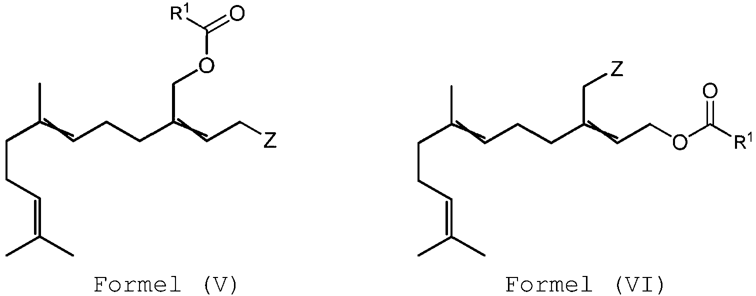
wobei das Verfahren den folgenden Schritt umfasst:
a) Inkontaktbringen einer Mischung, die eine Verbindung der Formel (III) und eine Verbindung der Formel (IV) umfasst, mit einem Acyliodid (R₁C(O)I) oder einem Acylhalogenid R₁C(O)X, wobei X aus Cl und Br ausgewählt ist, in Gegenwart von Iodsalz AI, wobei A aus Na, K, Li und quartären Ammoniumsalzen ausgewählt ist,
R¹ aus der Gruppe bestehend aus C₁- bis C₆-Alkyl ausgewählt ist und Z für Iod steht.

5. Verfahren nach einem der Ansprüche 1 bis 4, wobei es sich bei der Verbindung der Formel (III) und der Verbindung der Formel (IV) um die E-Formeln (III) und (IV) handelt:

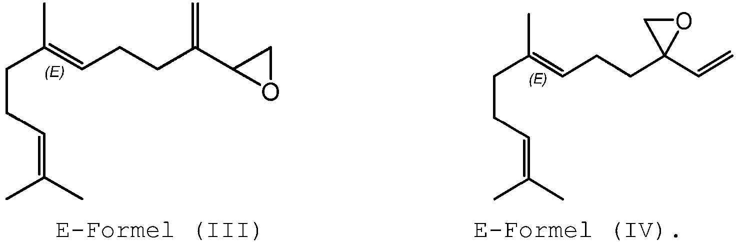

6. Verfahren nach einem der Ansprüche 1 bis 5, gefolgt von
Schritt b) Inkontaktbringen einer Verbindung der Formel (V) mit einer Verbindung der Formel (VII) 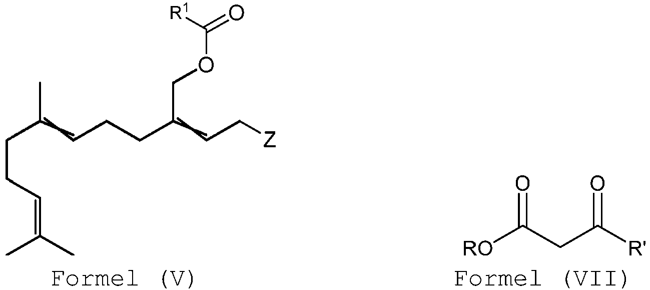 zum Erhalt einer Verbindung der Formel (VIII) 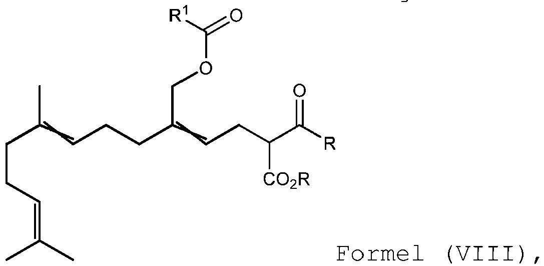 wobei R¹ aus der Gruppe bestehend aus C₁ bis C₆-Alkyl ausgewählt ist, R' aus der Gruppe bestehend aus einem C₁-C₆-Alkyl und einer OR-Gruppe ausgewählt ist, R aus der Gruppe bestehend aus Wasserstoff und einer C₁- bis C₆-Alkylgruppe ausgewählt ist und Z aus Cl, I und Br ausgewählt ist.

7. Verfahren nach Anspruch 6, gefolgt von
Schritt c) Hydrolyse und Decarboxylierung der Verbindung der Formel (VIII) 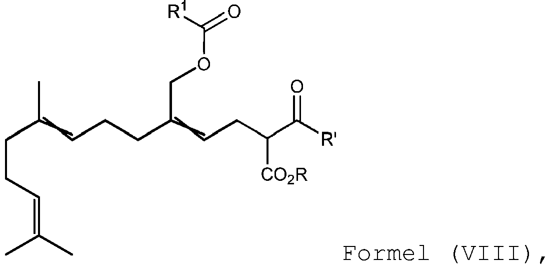 zum Erhalt einer Verbindung der Formel (I): 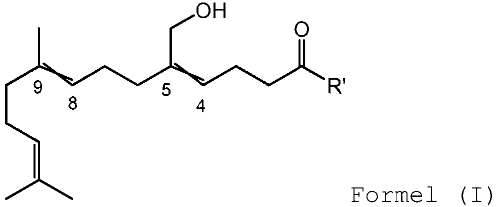 wobei R¹ aus der Gruppe bestehend aus C₁ bis C₆-Alkyl ausgewählt ist, R' aus der Gruppe bestehend aus einem C₁-C₆-Alkyl und einer OR-Gruppe ausgewählt ist und R aus der Gruppe bestehend aus Wasserstoff und einer C₁- bis C₆-Alkylgruppe ausgewählt ist.

8. Verfahren nach Anspruch 7, wobei der Schritt a) bis c) als eine sequentielle Eintopfsynthese zu verstehen ist.

9. Verfahren nach Anspruch 8, wobei der Schritt a) bis c) als eine sequentielle Eintopfsynthese ohne Isolierung von nicht umgesetztem Ausgangsstoff zu verstehen ist.

10. Verfahren nach Anspruch 8 oder Anspruch 9, wobei der Schritt a) bis c) als eine sequentielle Eintopfsynthese ohne Isolierung der Zwischenprodukte zu verstehen ist.

11. Verfahren nach Anspruch 7, wobei die in Schritt a) erhaltene Verbindung der Formel (V)
zuerst gereinigt wird, gefolgt von dem Verfahren, das die Schritte b) und c) umfasst, als sequentielle Eintopfsynthese.

12. Verfahren nach Anspruch 11, wobei es sich bei der Verbindung der Formel (V) um eine Verbindung der Formel (V') handelt. 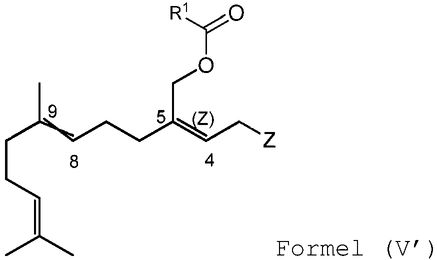 wobei
R¹ aus der Gruppe bestehend aus C₁- bis C₆-Alkyl ausgewählt ist und Z aus Cl und Br ausgewählt ist.

13. Verfahren nach einem der Ansprüche 1 bis 12, wobei R¹ für Methyl steht.

14. Verbindung der Formel (V) 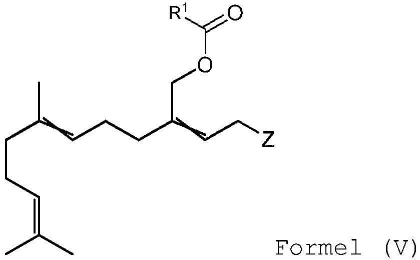 wobei
R¹ aus der Gruppe bestehend aus C₁- bis C₆-Alkyl ausgewählt ist und Z aus Cl, I und Br ausgewählt ist.

15. Verbindung der Formel (VI) 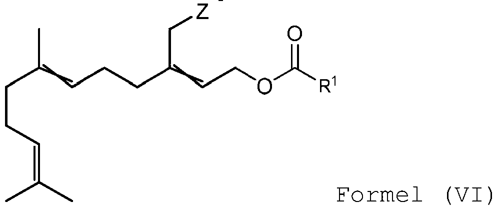 wobei
R¹ aus der Gruppe bestehend aus C₁- bis C₆-Alkyl ausgewählt ist und Z aus Cl, I und Br ausgewählt ist.

## Revendications

1. Procédé de préparation d'un composé de formule (V) 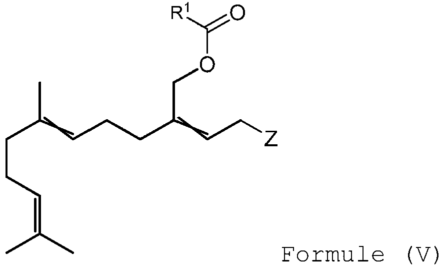
dans lequel le procédé comprend l'étape de
a) mise en contact d'un mélange comprenant un composé de formule (III) et un composé de formule (IV) avec un halogénure d'acyle R¹C(O)X éventuellement en présence de sel d'halogénure (AY) 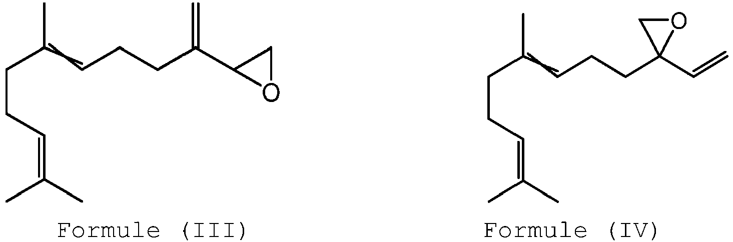
dans lequel R¹ est choisi dans le groupe constitué d'alkyle en C₁ à C₆, X, Y et Z sont choisis parmi Cl, I et Br, et A est choisi parmi Na, K, Li et des sels d'ammonium quaternaire.

2. Procédé selon la revendication 1 de préparation d'un composé de formule (V), dans lequel le composé de formule (V) est un composé de formule (V') 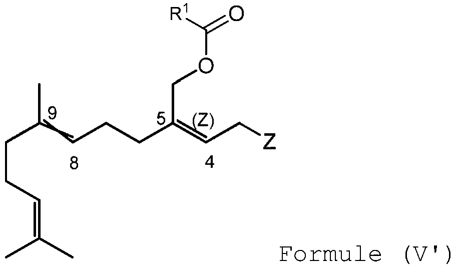
dans lequel le procédé comprend l'étape de
a) mise en contact d'un mélange comprenant un composé de formule (III) et un composé de formule (IV)
i) avec un halogénure d'acyle R¹C(O)X en l'absence d'un sel d'halogénure, ou
ii) avec un halogénure d'acyle R¹C(O)X en présence d'un sel d'halogénure AY dans lequel Y n'est pas iode,
dans lequel R¹ est choisi dans le groupe constitué d'alkyle en C₁ à C₆, X est choisi parmi Cl et Br, A est choisi parmi Na, K, Li et des sels d'ammonium quaternaire, et Z est choisi parmi Cl et Br.

3. Procédé selon la revendication 2 de préparation d'un composé de formule (V') et d'un composé de formule (X) et/ou son régioisomère (X') 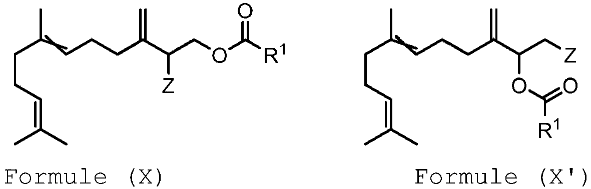 dans lequel R¹ est choisi dans le groupe constitué d'alkyle en C₁ à C₆ et Z est choisi parmi Cl et Br.

4. Procédé selon la revendication 1 de préparation d'un composé de formule (V) et d'un composé de formule (VI) 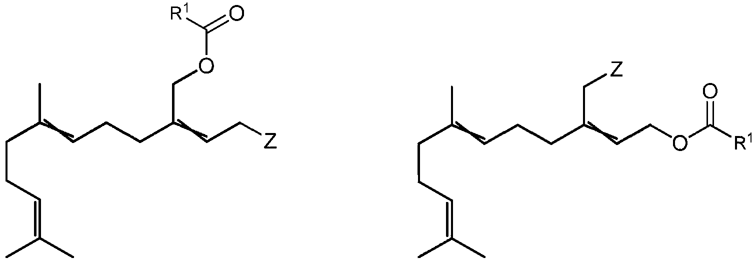 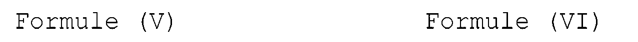
dans lequel le procédé comprend l'étape de
a) mise en contact d'un mélange comprenant un composé de formule (III) et un composé de formule (IV) avec un iodure d'acyle (R₁C(O)I), ou un halogénure d'acyle R₁C(O)X dans lequel X est choisi parmi Cl et Br en présence de sel d'iode AI dans lequel A est choisi parmi Na, K, Li et des sels d'ammonium quaternaire,
R¹ est choisi dans le groupe constitué d'alkyle en C₁ à C₆, et Z est iode.

5. Procédé selon l'une quelconque des revendications 1 à 4, dans lequel le composé de formule (III) et le composé de formule (IV) sont E-formule (III) et (IV)

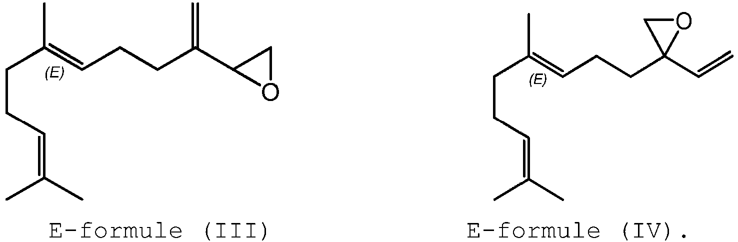

6. Procédé selon l'une quelconque des revendications 1 à 5, suivi par
l'étape de b) mise en contact d'un composé de formule (V) avec un composé de formule (VII) 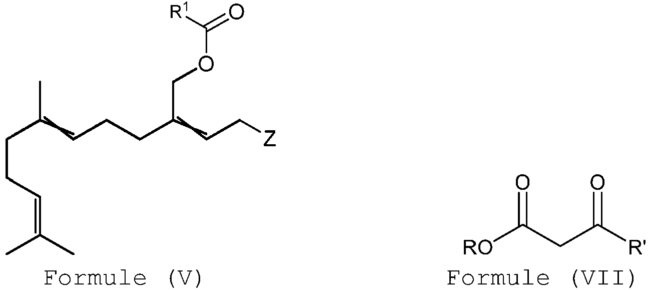 pour obtenir un composé de formule (VIII) 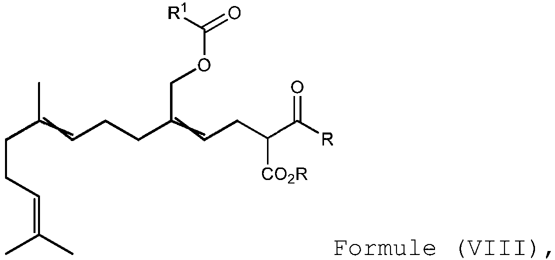 dans lequel R¹ est choisi dans le groupe constitué d'alkyle en C₁ à C₆, R' est choisi dans le groupe constitué d'alkyle en C₁ à C₆ et d'un groupe OR, R est choisi dans le groupe constitué d'hydrogène et d'un groupe alkyle en C₁ à C₆, et Z est choisi parmi Cl, I et Br.

7. Procédé selon la revendication 6, suivi par
l'étape c) d'hydrolyse et de décarboxylation du composé de formule (VIII) 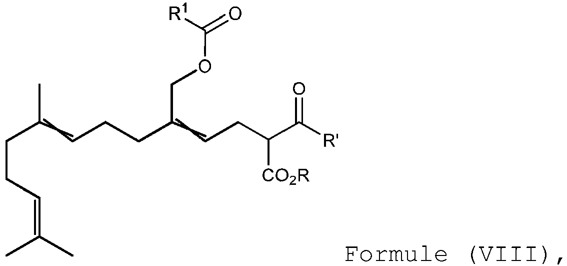 pour obtenir un composé de formule (I) 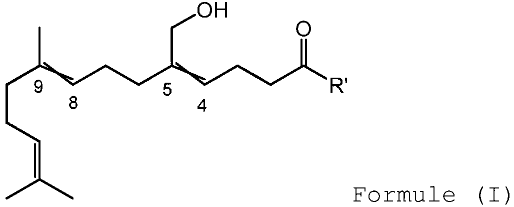 dans lequel R¹ est choisi dans le groupe constitué d'alkyle en C₁ à C₆, R' est choisi dans le groupe constitué d'un alkyle en C₁ à C₆ et d'un groupe OR, et R est choisi dans le groupe constitué d'hydrogène et d'un groupe alkyle en C₁ à C₆.

8. Procédé selon la revendication 7, dans lequel les étapes a) à c) doivent être comprises comme une synthèse séquentielle dans un pot.

9. Procédé selon la revendication 8, dans lequel les étapes a) à c) doivent être comprises comme une synthèse séquentielle dans un pot sans isolement de la matière de départ n'ayant pas réagi.

10. Procédé selon la revendication 8 ou la revendication 9, dans lequel les étapes a) à c) doivent être comprises comme une synthèse séquentielle dans un pot sans isolement des intermédiaires.

11. Procédé selon la revendication 7, dans lequel le composé de formule (V) obtenu dans l'étape a) est d'abord purifié, suivi du procédé comprenant les étapes b) et c) en tant que synthèse séquentielle dans un pot.

12. Procédé selon la revendication 11, dans lequel le composé de formule (V) est un composé de formule (V') 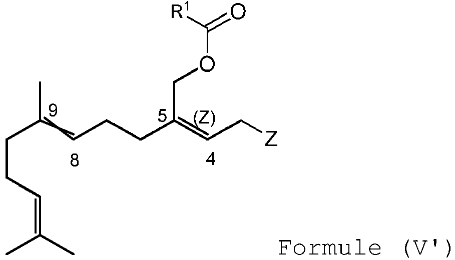 dans lequel
R¹ est choisi dans le groupe constitué d'alkyle en C₁ à C₆, et Z est choisi parmi Cl et Br.

13. Procédé selon l'une quelconque des revendications 1 à 12, dans lequel R¹ est méthyle.

14. Composé de formule (V) 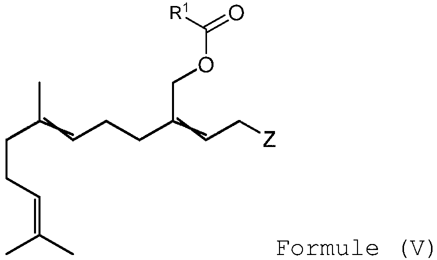 dans lequel
R¹ est choisi dans le groupe constitué par alkyle en C₁ à C₆ et Z est choisi parmi Cl, I et Br.

15. Composé de formule (VI) 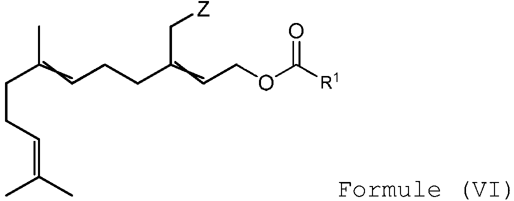 dans lequel
R¹ est choisi dans le groupe constitué par alkyle en C₁ à C₆ et Z est choisi parmi Cl, I et Br.
